# EUROPEAN PATENT SPECIFICATION

(11) **EP 4 475 761 B1**
(45) Date of publication and mention of the grant of the patent: **16.07.2025**
(21) Application number: 23711206.5
(22) Date of filing: 05.02.2023
(51) Int. Cl.: A61B 5/1459

(54) **MEDICAL IMPLANT DEVICE**
MEDIZINISCHE IMPLANTATVORRICHTUNG
DISPOSITIF D'IMPLANT MÉDICAL

(30) Priority: 09.02.2022 US 202263308460 P
(43) Date of publication of application: 18.12.2024
(73) Proprietor: Edwards Lifesciences Corporation, Irvine, CA 92614 (US)
(72) Inventor: KARAPETIAN, Emil, Irvine, CA 92614 (US); CHITSAZ, Sam, Sacramento, CA 95814 (US)
(74) Representative: Eisenführ Speiser
(86) International application number: PCT/US2023/012366
(87) International publication number: WO 2023/154236

(56) References cited:
- EP-A1- 1 386 637
- US-A- 5 199 428
- US-A1- 2014 107 722
- US-B1- 8 175 668
- US-B2- 8 929 982

## Description

### BACKGROUND

The present disclosure generally relates to the field of measuring light absorption properties of blood flowing through a coronary vein. Deoxygenated blood from the myocardium can drain into the coronary veins, including the coronary sinus, which can then return the deoxygenated blood to the right atrium. Measuring the oxygenation of the blood flowing through a coronary vein can be used in monitoring various health metrics, including monitoring health of the heart.

US 8,175,668 B1 describes an intravenous implantable optical sensor which assesses the relative absorbance of multiple wavelengths of light in order to determine oxygen saturation. The calculation of oxygen saturation is enhanced by use of a function of hematocrit which is derived from the relative absorbance of light of an isobestic wavelength along two different length paths through the blood. The use of the hematocrit-dependent term and multiple wavelengths of light to calculate oxygen saturation provides results that are less susceptible to noise and variation in hematocrit and thus provides a more accurate measure of oxygen saturation over a wider range of conditions than previously possible. The optical sensor may form part of an implantable system which performs the calculation of oxygen saturation and uses the results for a diagnostic or therapeutic purpose.

### SUMMARY

The claimed invention relates to a first medical implant device as defined in independent claim 1. Some preferred configurations of the first medical implant device are defined in dependent claims 2 to 10. The claimed invention further relates to a second medical implant device as defined in independent claim 11. Some preferred configurations of the second medical implant device are defined in dependent claims 12 to 15.

Also described herein are related aspects, examples, embodiments and arrangements useful for understanding the claimed invention, and which do not necessarily constitute embodiments of the claimed invention. The subject-matter for which protection is sought is defined by the claims.

Described herein are methods and devices related to a medical implant device configured to provide light absorption information of blood flowing through a coronary vein from a position external of the coronary vein. For example, the implant device can be configured to provide light absorption information of blood flowing through a coronary sinus from a position external of the coronary sinus. The medical implant device can comprise a housing, and a light source and a light sensor received in the housing. The housing can be configured to be positioned externally of the coronary vein. For example, the housing receiving the light source and light sensor can be positioned externally of the coronary sinus, including over an external surface of a coronary sinus wall and/or over a surface of a left atrial wall portion adjacent to a portion of the coronary sinus wall. The light absorption measurements can be used to determine an oxygen saturation level of the blood flowing through the coronary vein, such as the coronary sinus.

Methods and structures disclosed herein for treating a patient also encompass analogous methods and structures performed on or placed on a simulated patient, which is useful, for example, for training; for demonstration; for procedure and/or device development; and the like. The simulated patient can be physical, virtual, or a combination of physical and virtual. A simulation can include a simulation of all or a portion of a patient, for example, an entire body, a portion of a body (*e.g.,* thorax), a system (*e.g.,* cardiovascular system), an organ (*e.g.,* heart), or any combination thereof. Physical elements can be natural, including human or animal cadavers, or portions thereof; synthetic; or any combination of natural and synthetic. Virtual elements can be entirely in silica, or overlaid on one or more of the physical components. Virtual elements can be presented on any combination of screens, headsets, holographically, projected, loud speakers, headphones, pressure transducers, temperature transducers, or using any combination of suitable technologies.

For purposes of summarizing the disclosure, certain aspects, advantages and novel features have been described herein. It is to be understood that not necessarily all such advantages may be achieved in accordance with any particular example. Thus, the disclosed examples may be carried out in a manner that achieves or optimizes one advantage or group of advantages as taught herein without necessarily achieving other advantages as may be taught or suggested herein.

### BRIEF DESCRIPTION OF THE DRAWINGS

Various examples are depicted in the accompanying drawings for illustrative purposes and should in no way be interpreted as limiting. In addition, various features of different disclosed examples can be combined to form additional examples, which are part of this disclosure. Throughout the drawings, reference numbers may be reused to indicate correspondence between reference elements. However, it should be understood that the use of similar reference numbers in connection with multiple drawings does not necessarily imply similarity between respective examples associated therewith. Furthermore, it should be understood that the features of the respective drawings are not necessarily drawn to scale, and the illustrated sizes thereof are presented for the purpose of illustration of aspects thereof. Generally, certain of the illustrated features may be relatively smaller than as illustrated in some examples or configurations.
Figure 1 is a cross-sectional view of a human heart.
Figures 2 is a side perspective view of the human heart, and a medical implant device positioned over an extraluminal surface of a coronary sinus of the heart.
Figures 3 is a side cross-sectional view of an example of a medical implant device positioned over the extraluminal surface of the coronary sinus.
Figure 4A is a perspective view, and Figure 4B is a side cross-sectional view, of the medical implant device of Figure 3.
Figure 5A is a perspective view, and Figure 5B is a side cross-sectional view, of another example of a medical implant device configured to be positioned over the extraluminal surface of the coronary sinus.
Figure 6 is a side cross-sectional view of yet another example of a medical implant device positioned over the extraluminal surface of the coronary sinus.
Figure 7A is a perspective view, and Figure 7B is a side cross-sectional view, of the medical implant device of Figure 6.
Figure 8 is a side cross-sectional view of an example of a medical implant device comprising a magnetically coupled anchor, where a portion of the medical implant device is positioned over the extraluminal surface of the coronary sinus and another portion of the medical implant device is positioned within the coronary sinus.
Figure 9A is a perspective view, and Figure 9B is a side cross-sectional view, of the portion of the medical implant device of Figure 8 configured to be positioned over an extraluminal surface of a coronary sinus. Figure 9C is a perspective view, and Figure 9D is a side cross-sectional view, of the portion of the medical implant device configured to be positioned within the coronary sinus.
Figure 10A is a perspective view, and Figure 10B is a side cross-sectional view, of a portion of another example of a medical implant device comprising a magnetic anchoring mechanism that is configured to be positioned over an extraluminal surface of a coronary sinus. Figure 10C is a perspective view, and Figure 10D is a side cross-sectional view, of a portion of the medical implant device configured to be positioned within the coronary sinus.
Figure 11 shows a medical implant device positioned within the left atrium and over a left atrial wall portion adjacent to a portion of the coronary sinus.
Figure 12A is a perspective view, and Figure 12B is a side cross-sectional view, of the medical implant device of Figure 11.
Figure 13 is a flow diagram of an example of a process for measuring light absorption properties of blood flowing through the coronary sinus using a medical implant device as described herein.

### DETAILED DESCRIPTION

The headings provided herein are for convenience only and do not necessarily affect the scope or meaning of the claimed invention, which is defined in the independent claims.

The present disclosure provides systems, devices, and methods relating to a medical implant device configured to measure light absorption properties of blood flowing through a coronary vein, including a coronary sinus, for determining oxygen saturation of the blood. A light source and a light sensor of the medical implant device can be positioned over a portion of the heart external of the coronary vein, such as the coronary sinus, to provide the light absorption measurements. For example, the light source and a light sensor can be positioned over an external surface of a coronary sinus wall and/or over a surface of a left atrial wall portion adjacent to a portion of the coronary sinus wall.

In any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain examples; however, the order of description should not be construed to imply that these operations are order dependent. Additionally, the structures, systems, and/or devices described herein may be embodied as integrated components or as separate components. For purposes of comparing various examples, certain aspects and advantages of these examples are described. Not necessarily all such aspects or advantages are achieved by any particular example. Thus, for example, various examples may be carried out in a manner that achieves or optimizes one advantage or group of advantages as taught herein without necessarily achieving other aspects or advantages as may also be taught or suggested herein.

Certain standard anatomical terms of location are used herein to refer to the anatomy of animals, and namely humans, with respect to the preferred examples. Although certain spatially relative terms, such as "outer," "inner," "upper," "lower," "below," "above," "vertical," "horizontal," "top," "bottom," and similar terms, are used herein to describe a spatial relationship of one device/element or anatomical structure to another device/element or anatomical structure, it is understood that these terms are used herein for ease of description to describe the positional relationship between element(s)/structures(s), as illustrated in the drawings. It should be understood that spatially relative terms are intended to encompass different orientations of the element(s)/structures(s), in use or operation, in addition to the orientations depicted in the drawings. For example, an element/structure described as "above" another element/structure may represent a position that is below or beside such other element/structure with respect to alternate orientations of the subject patient or element/structure, and vice-versa.

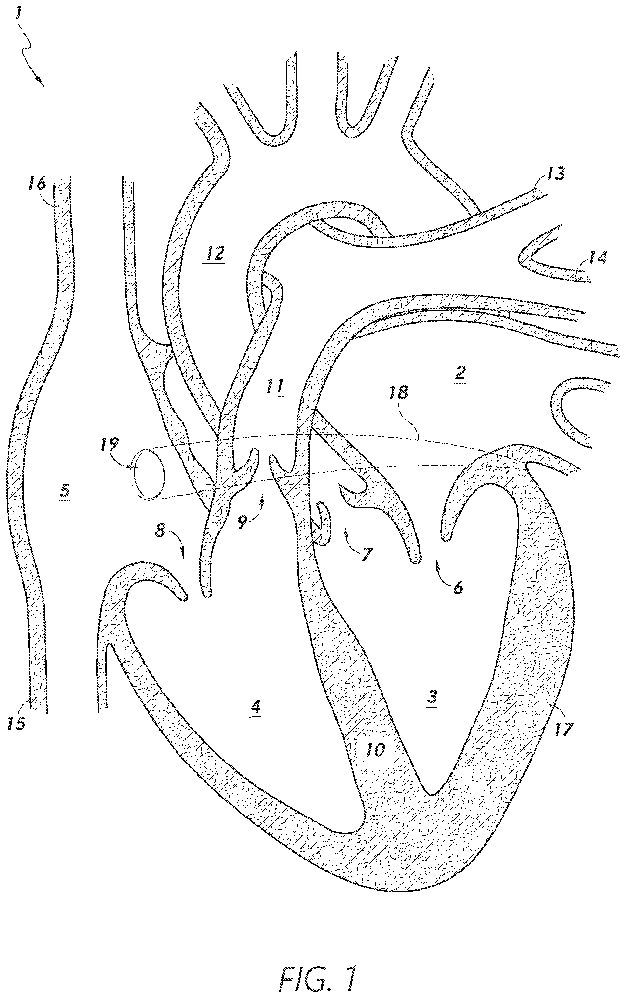

Figure 1 is a schematic diagram showing various features of a human heart 1. The heart 1 includes four chambers within a heart wall 17, namely the left atrium 2, the left ventricle 3, the right ventricle 4, and the right atrium 5. A wall of muscle, referred to as the septum 10, separates the left atrium 2 and right atrium 5, and the left ventricle 3 and right ventricle 4. Blood flow through the heart 1 is at least partially controlled by four valves, the mitral valve 6, aortic valve 7, tricuspid valve 8, and pulmonary valve 9. The mitral valve 6 separates the left atrium 2 and the left ventricle 3 and controls blood flow therebetween. The aortic valve 7 separates and controls blood flow between the left ventricle 3 and the aorta 12. The tricuspid valve 8 separates the right atrium 5 and the right ventricle 4 and controls blood flow therebetween. The pulmonary valve 9 separates the right ventricle 4 and the pulmonary trunk or artery 11, controlling blood flow therebetween.

In a healthy heart, the heart can receive deoxygenated blood arriving from the rest of the body generally into the right side of the heart for transport to the lungs, and oxygenated blood from the lungs generally into the left side of the heart for transport to the rest of the body. During ventricular diastole, deoxygenated blood arrive in the right atrium 5 from the inferior vena cava 15 and superior vena cava 16 to flow into the right ventricle 4, and oxygenated blood arrive in the left atrium 2 from the pulmonary veins to flow into the left ventricle 3. During ventricular systole, deoxygenated blood from the right ventricle 4 can flow into the pulmonary trunk 11 for transport to the lungs (*e.g.,* via the left 14 and right 13 pulmonary arteries), and oxygenated blood can flow from the left ventricle 3 to the aorta 12 for transport to the rest of the body.

Veins collecting deoxygenated blood from the myocardium can drain into the coronary sinus 18. The coronary sinus 18 can then return the deoxygenated blood into the right atrium 5 through the coronary sinus ostium 19. Coronary vein blood oxygen saturation information, such as coronary sinus blood oxygen saturation information, allows monitoring of oxygen utilization by the myocardium. Determining oxygen saturation (*e.g*., SaO₂) of blood flowing through a coronary vein, such as the coronary sinus 18, can provide information regarding the health of the heart 1. Oxygen saturation of blood flowing through the coronary vein, such as the coronary sinus 18, can directly provide cardiac oxygen utilization data, including changes in the cardiac oxygen utilization. Cardiac oxygen utilization data and/or changes in cardiac oxygen utilization data can be used to detect and/or track the progress in inefficient myocardial oxygenation. In some instances, such data can be used to predict acute decompensated heart failure (ADHF), thereby providing an opportunity for preventative measures. The ability to take preventative measures can prevent or reduce hospital admissions and/or unscheduled medical interventions, improving outcomes for patients.

Oxygenated hemoglobin and deoxygenated hemoglobin can have different light absorption properties, for example demonstrating different ability to absorb light at certain wavelengths. Oxygenated hemoglobin and deoxygenated hemoglobin can absorb infrared light and red light differently. Oxygenated hemoglobin can absorb more infrared light than red light, for example reflecting more red light than infrared light. Deoxygenated hemoglobin can absorb more red light than infrared light, for example reflecting more infrared light than red light. The difference in light absorption properties of oxygenated and deoxygenated hemoglobin can be used to determine blood oxygen saturation information for the blood flowing through the coronary vein, including the coronary sinus.

The disclosure herein provides one or more devices and methods related a medical implant device configured to provide light absorption information of blood flowing through a coronary vein, including a coronary sinus. For example, the medical implant device can comprise a housing, and a light source and a light sensor received in the housing, where the housing is configured to be positioned externally of the coronary sinus. The light source and light sensor can provide measurements of light absorption properties for blood flowing through the coronary sinus from a location external of the coronary sinus lumen. The housing can be positioned over an external surface of a coronary sinus wall and/or over a surface of a heart wall portion adjacent to a portion of the coronary sinus wall. The medical implant device can comprise an anchor configured to mechanically and/or magnetically secure the housing over the external surface of the coronary sinus wall or the surface of the heart wall portion adjacent to the coronary sinus.

In some instances, a first wall portion of the housing can be positioned over an external surface of a coronary sinus wall. For example, the first wall portion can be over an extraluminal surface of the coronary sinus. Light signals emitted from the light source can be pass through a first light-transparent region on the first wall portion. The emitted light signals can be transmitted through the coronary sinus wall and into the coronary sinus. Portions of the light signals transmitted into the coronary sinus not absorbed by the blood flowing through the coronary sinus can be reflected back through the coronary sinus wall and pass through a second light-transparent region on the first wall portion for detection by the light sensor. In some instances, the first and second light-transparent regions may not be distinct regions. For example, light signals emitted from the light source and light signals reflected from the coronary sinus can pass through the same light-transparent region on the first wall portion.

In some instances, a first wall portion of the housing can be positioned over an atrial wall portion adjacent to a portion of the coronary sinus wall. For example, the housing can be positioned within the left atrium. As described herein, a portion of the coronary sinus can extend over an external surface of the left atrium. The first wall portion of the medical implant device can be configured to be positioned over a left atrial wall portion adjacent to a portion of the coronary sinus wall, such as a surface of the atrial wall portion oriented toward the left atrium. The light sensor can be configured to emit light signals that pass through the first light-transparent region of the first wall portion. The emitted light signals can travel through the left atrial wall portion and the adjacent portion of the coronary sinus wall, and into the coronary sinus. Portions of the light signals transmitted into the coronary sinus not absorbed by the blood flowing through the coronary sinus can be reflected back through the coronary sinus wall and the adjacent portion of the left atrial wall. The reflected light signals can pass through the second light-transparent region of the first wall portion for detection by the light sensor.

The light source can be configured to emit light signals at one or more wavelengths at which oxygenated and deoxygenated hemoglobin demonstrate differentiable light absorption properties. The light sensor can be configured to detect light signals at these one or more wavelengths. In some instances, the light source can be configured to emit a first light signal at a first wavelength and a second light signal at a second wavelength. In some instances, the first light signal can be an infrared light signal, such as a light signal having a wavelength of about 660 nanometer (nm). The second light signal can be a red light signal, such as a light signal having a wavelength of about 940 nanometers (nm). The light sensor can be configured to detect reflected portions of the infrared and red light signals that had been emitted into the coronary sinus.

Emission of light signals by the light source and detection of reflected light signals by the light sensor can occur at any number of different time intervals. In some instances, the light source and light sensor can be configured to continuously emit light and detect light for continuous monitoring of oxygen saturation of blood flowing through the coronary sinus. In some instances, the light source and light sensor can be configured to emit light and detect light at predetermined time intervals. For example, the light source and light sensor can be configured to emit and detect light for a predetermined period every hour, day, or week. In some instances, the light source and light sensor can be triggered and/or activated by an external stimulus. The external stimulus can be generated by an operator and/or auto-generated at a preset time interval. For example, the light source and light sensor can be turned on for on-demand measurements.

Monitoring of oxygen utilization by the myocardium can facilitate detection of a number of conditions. A drop in blood oxygen saturation can be due to insufficient coronary blood perfusion, indicating for example, epicardial coronary stenosis and/or coronary microvascular disease. A decrease in blood oxygen saturation can also be due to demand ischemia, such as higher oxygen demand than available oxygen supply. A high oxygen demand relative to oxygen supply can indicate hypertrophic cardiomyopathy, and/or tachycardia. Increase in blood oxygen saturation can indicate, for example, energy metabolism impairment in heart failure with preserved ejection fraction (HFpEF), myocardial fibrosis, scar formation and/or infiltrative disorders.

In some instances, coronary vein blood oxygen saturation, such as coronary sinus blood oxygen saturation, can be used in combination with other physiological metrics to monitor the physiological state of the patient, such as to improve tracking of disease progress and/or diagnosis. In some instances, coronary vein blood oxygen saturation, including coronary sinus blood oxygen saturation, can be used in combination with hemodynamic metrics (*e.g.,* intracardiac pressures, systemic blood pressure, heart rate), electrophysiology measurements (*e.g.,* EKG), systemic oxygen saturation (*e.g.,* systemic SaO2), venous oxygen saturation (SvO2), and/or indices of aerobic and anaerobic metabolism (*e.g.,* pH, lactate).

In some instances, a measurement of a systemic arterial blood oxygen saturation can be used in combination with a measurement of a coronary vein blood oxygen saturation, such as a coronary sinus blood oxygen saturation, to facilitate determining the health of the heart, including prediction of acute decompensated heart failure (ADHF). One or more systemic arterial blood oxygen saturation measurements be made to provide a blood oxygen saturation baseline for a patient, for example serving as a reference point against which coronary vein blood oxygen saturation measurements, such as coronary sinus blood oxygen saturation measurements, can be compared in determining whether the coronary vein, including coronary sinus, blood oxygen saturation measurements indicate a problem with heart function or an oxygenation problem elsewhere in the body (*e.g.,* a deterioration of blood oxygenation in the lungs). A difference between the systemic arterial blood oxygen saturation and coronary vein blood oxygen saturation, including the coronary sinus blood oxygen saturation, can be determined. For example, the difference can be determined such that a change in the difference between the systemic arterial blood oxygen saturation and the coronary vein or the coronary sinus blood oxygen saturation, in combination with a change in the coronary vein or coronary sinus blood oxygen saturation, can indicate deteriorating heart function.

The medical implant device can comprise one or more components for performing pulse oximetry. For example, the medical implant device can comprise the light source and light sensor, and one or more other components of a pulse oximeter. In some instances, the housing of the medical implant device can be configured to receive the light source and light sensor, and the one or more other components of the pulse oximeter. In some instances, the medical implant device can comprise a pulse oximeter. In some instances, the medical implant device can comprise a reflective pulse oximeter. For example, the housing can be configured to receive all of the components of a pulse oximeter. In some instances, the light source and light sensor can be received in the housing while other components for determining the oxygen saturation information can be externally positioned. For example, measurements for light absorption properties made by the medical implant device can be communicated, transmit and/or transferred, such as by any number of wireless communication methods, to a device external of the patient such that the oxygen saturation metric can be calculated.

In some instances, the medical implant device can be powered using wireless charging, including inductive charging. For example, the medical implant device can comprise one or more batteries configured to be charged inductively. For example, the patient can be positioned over and/or in contact with an inductive charging plate for charging of the one or more batteries.

Although description herein refers primarily to the coronary sinus, it will be understood that one or more other coronary veins can be applicable. For example, one or more medical implant devices described herein can be configured to provide light absorption information of blood flowing through another coronary vein of the greater cardiac venous system, such as the great cardiac vein. In some instances, the first wall portion of the housing receiving the light source and light sensor can be positioned over an extraluminal surface of the great cardiac vein. In some instances, the first wall portion of the housing can be positioned over an atrial wall portion adjacent to a portion of a wall of the great cardiac vein. In some instances, one or more medical implant devices described herein can be configured to provide light absorption information of blood flowing through one or more of a middle cardiac vein, small cardiac vein, right marginal vein, left marginal vein, anterior cardiac vein, left and right ventricular veins, left and right atrial veins, inferior vein of the left ventricle and/or oblique vein of the left atrium. In some instances, light absorption information can be combined from multiple measurement locations. For example, each of multiple medical implant devices as described herein can be positioned at a respective location for measuring light absorption information of blood flowing through a respective coronary vein.

Any of the various systems, devices, apparatuses, etc. in this disclosure can be sterilized (*e.g.*, with heat, radiation, ethylene oxide, hydrogen peroxide, etc.) to ensure they are safe for use with patients, and the methods herein can comprise sterilization of the associated system, device, apparatus, etc. *(e.g.,* with heat, radiation, ethylene oxide, hydrogen peroxide, etc.).

The term "associated with" is used herein according to its broad and ordinary meaning. For example, where a first feature, element, component, device, or member is described as being "associated with" a second feature, element, component, device, or member, such description should be understood as indicating that the first feature, element, component, device, or member is physically coupled, attached, or connected to, integrated with, embedded at least partially within, or otherwise physically related to the second feature, element, component, device, or member, whether directly or indirectly.

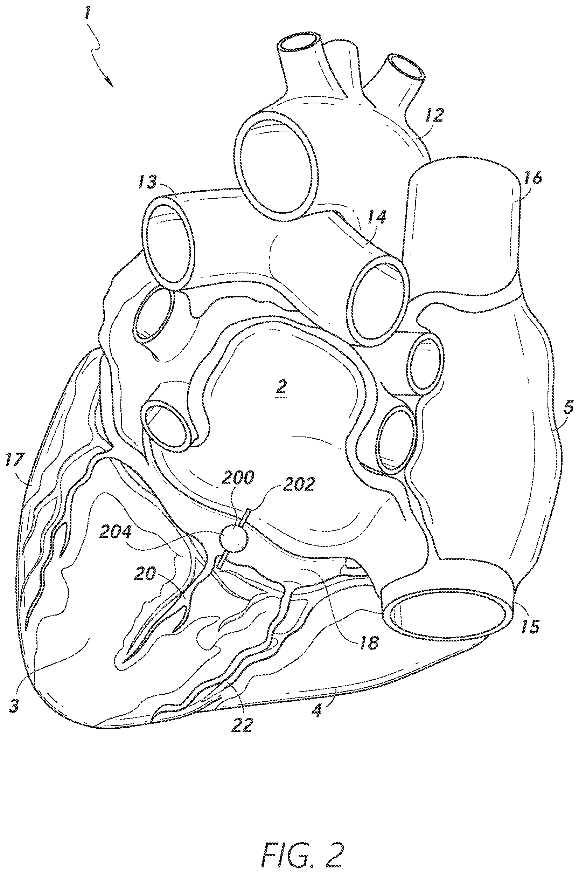

Referring to Figure 2, a perspective view of an exterior of a human heart is shown, and a medical implant device 200 is shown as being positioned over an extraluminal surface of a coronary sinus 18 of the heart 1. Various veins 20 that collect deoxygenated blood from the myocardium and drain into the coronary sinus 19 are shown in the figure. As described in further detail herein, the medical implant device 200 can comprise a housing 204 configured to receive a light source and a light sensor. The light source can be configured to emit light signals at one or more wavelengths such that the emitted light signals can pass through the coronary sinus wall and be transmitted into the coronary sinus 18. The light sensor can be configured to detect light signals at the one or more wavelengths. For example, the light sensor can be configured to detect portions of the light signals transmitted into the coronary sinus 18 that are not absorbed and are reflected by the blood flowing through the coronary sinus 18.

The medical implant device 200 can comprise an anchor 202 associated with the housing 204 to secure the medical implant device 200 to the heart. The anchor 202 can be configured to position a portion of the housing 204 over and/or in contact with the extraluminal surface of the coronary sinus 18 to facilitate desired transmission of light signals into the coronary sinus 18 and detection of reflected light signals from the coronary sinus 18. As described in further detail herein, the anchor 202 can be configured to be secured to one or more adjacent portions of the heart wall 17 on either side of the coronary sinus 18.

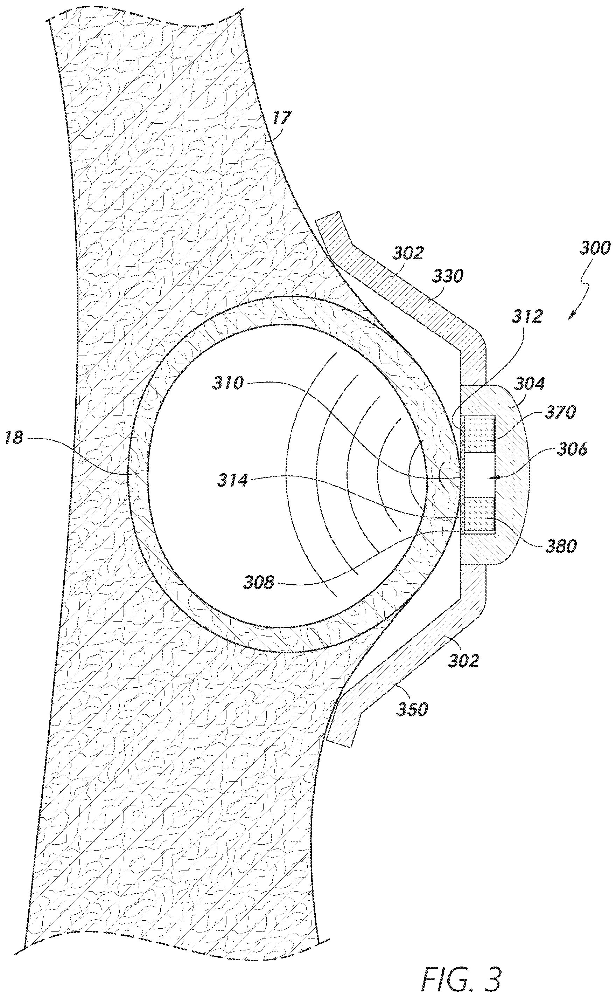

Figure 3 is a side cross-sectional view of an example of a medical implant device 300 configured to be positioned over an extraluminal surface of a coronary sinus 18. Figure 3 shows the medical implant device 300 positioned over the extraluminal surface of the coronary sinus 18. The medical implant device 300 can comprise a light source 370 and a light sensor 380 configured to provide measurements of light absorption properties of blood flowing through the coronary sinus 18. The light source 370 and light sensor 380 can be received within a cavity 306 of a housing 304 that is configured to be positioned over the extraluminal surface. For example, at least a portion of a first wall portion 308 of the housing 304 can be configured to be positioned over the extraluminal surface of the coronary sinus 18. The first wall portion 308 can comprise a first surface 310 configured to be oriented toward and/or in contact with the extraluminal surface. A first light-transparent region 312 of the first wall portion 308 can be configured to allow passage therethrough of emitted light signals from the light source 370. The emitted light signal can pass through the first light-transparent region 312, and the portion of the coronary sinus wall over which the housing 304 is positioned and into the coronary sinus 18. A second light-transparent region 314 of the first wall portion 308 can be configured to allow passage therethrough of reflected light signals from within the coronary sinus 18 for detection by the light sensor 380. For example, portions of the emitted light signals not absorbed by the blood flowing through the coronary sinus 18 can be reflected back through the coronary sinus wall for detection by the light sensor 380. In some instances, the first and second light-transparent regions 312, 314 may not be distinct regions. In some instances, the first and second light-transparent regions 312, 314 can at least partially overlap. In some instances, the first and second light-transparent regions 312, 314 can comprise a common light-transparent region. For example, light signals emitted from the light source 370 for transmission into the coronary sinus 18 and light signals reflected from the coronary sinus 18 for detection by the light sensor 380 can pass through the same light-transparent region on the first wall portion 308.

An anchor 302 can be associated with, for example coupled to, the housing 304 and configured to secure the medical implant device 300 to the heart. The anchor 302 can be configured to mechanically couple the medical implant device 300 to one or more heart wall portions adjacent to the coronary sinus 18. For example, the anchor 302 can comprise a first anchor component 330 and a second anchor component 350 each comprising a portion configured to be secured to portions of the heart wall 17 on either side of the coronary sinus 18. The first anchor component 330 and a second anchor component 350 can secure the implant device 300 to the heart wall 17 such that the first wall portion 308 of the housing 304 can be positioned over and/or in contact with a portion of the extraluminal surface of the coronary sinus 18. The first wall portion 308 can be positioned over and/or in contact with the extraluminal surface of the coronary sinus 18 to facilitate transmitting light signals into and receiving light signals from the coronary sinus 18. It will be understood that although the medical implant device 300 can comprise two anchor components, fewer or more anchor components can be applicable. For example, in some instances, a medical implant device can comprise one anchor component. The one anchor component can be configured to be secured to a heart wall portion adjacent to the coronary sinus. In some instances, a medical implant device can comprise three or four anchor components.

In some instances, the medical implant device 300 can be powered using inductive charging. One or more batteries can be associated with one or more portions of the medical implant device 300, for example being received within the cavity 306 of the housing 304. The one or more batteries can be charged inductively. For example, the patient can be positioned over and/or in contact with an inductive charging plate such that the one or more batteries can be charged.

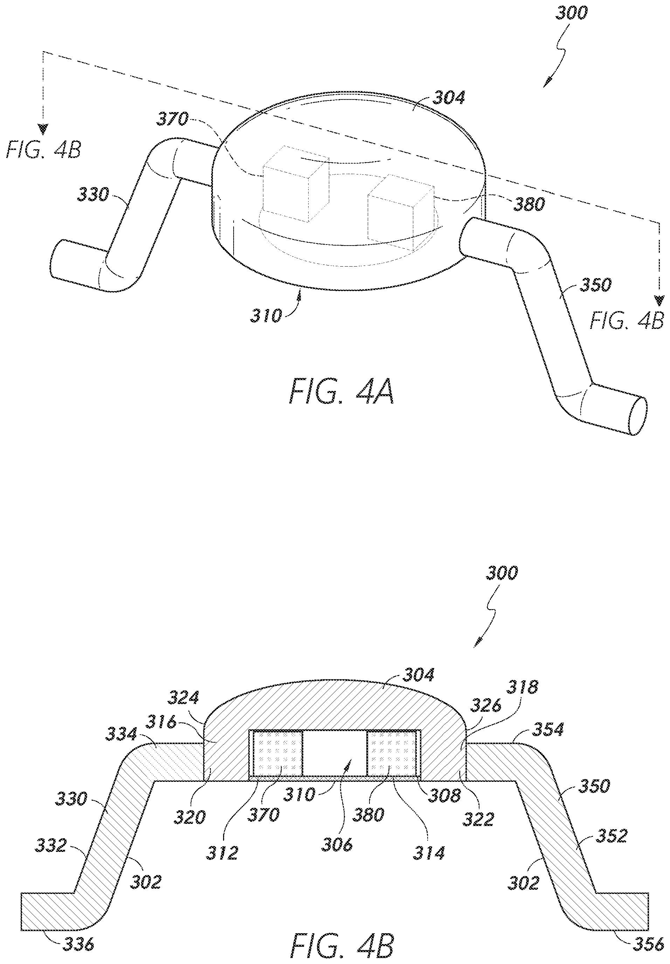

Figure 4A is a perspective view, and Figure 4B is a side cross-sectional view, of the medical implant device 300 described with reference to Figure 3. The first and second anchor components 330, 350 can each comprise a respective portion having an angled orientation relative to the first wall portion 308, such as relative to the first surface 310, of the housing 304. The respective portions of the first and second anchor components 330, 350 having the angled orientation can comprise at least a portion that extends between the housing 304 and the heart wall, such that the first and second anchor components 330, 350 can facilitate securing the medical implant device 300 to a heart wall portion adjacent to a coronary sinus.

The first and second anchor components 330, 350 can be coupled to portions of the housing 304 such that the anchor components 330, 350 do not interfere with emission of light signals by the light source 370 into the coronary sinus and detection by the light sensor 380 of reflected light signals from the coronary sinus. The first anchor component 330 can be coupled to a first housing portion 316. The second anchor component 350 can be coupled to a second housing portion 318. The first and second housing portions 316, 318 can be laterally disposed from the first and second light-transparent regions 312, 314. In some instances, the first housing portion 316 can comprise a first lateral wall portion 320 and the second housing portion 318 can comprise a second lateral wall portion 322. In some instances, the first and second lateral housing portions 320, 322 can be at an angle relative to the first wall portion 308. In some instances, the first and second lateral housing portions 320, 322 can have an orientation perpendicular or substantially perpendicular to that of the first wall portion 308. For example, the first lateral wall portion 320 can comprise a first external lateral surface 324 to which the first anchor component 330 can be coupled. The second lateral wall portion 322 can comprise a second external lateral surface 326 to which the second anchor component 350 can be coupled. In some instances, the first housing portion 316 and the second housing portion 318 can be at opposing positions on the housing 304. The first external surface 324 and the second external surface 326 can each have a perpendicular or substantially perpendicular orientation relative to the first wall portion 308, including the first surface 310 of the first wall portion 308.

The first anchor component 330 can comprise a first anchor portion 332 oriented at an angle relative to the first wall portion 308, including the first surface 310. The second anchor component 350 can comprise a second anchor portion 352 oriented at an angle relative to the first wall portion 308, including the first surface 310. A first end portion 334, 354 of each of the first and second anchor components 330, 350 can be coupled to the respective housing portions. For example, the first end portion 334 of the first anchor component 330 can be coupled to the first lateral wall portion 320, including the first external lateral surface 324. The first end portion 354 of the second anchor component 350 can be coupled to the second lateral wall portion 322, including the second external lateral surface 326. In some instances, the first end portion 334 of the first anchor component 330 can comprise a lateral anchor portion coupled to the first lateral wall portion 320. The first end portion 354 of the second anchor component 350 can comprise a second lateral anchor portion coupled to the second lateral wall portion 322. Each of the first lateral anchor portion and the second lateral anchor portion can have the same orientation as the first surface 310 of the first wall portion 308. A second end portion 336, 356 of each of the first and second anchor components 330, 350 can be configured to be coupled to respective heart wall portions. For example, the second end portion 336 of the first anchor component 330 can be secured to a first heart wall portion on a first side of the coronary sinus. The second end portion 356 of the second anchor component 350 can be secured to a second heart wall portion on a second side of the coronary sinus. In some instances, each of the second end portions 336, 356 can be mechanically secured to the heart wall. In some instances, each of the second end portions 336, 356 can comprise at least one of a clip, corkscrew and barb. In some instances, each of the second end portions 336, 356 can comprise openings configured to facilitate stitching of the second end portions 336, 356 to the heart wall. For example, the openings can receive a stitch, suture, cord and/or other fastener that is configured to be secured to the heart wall.

In some instances, the light source 370 can comprise one or more light emitting diodes (LEDs). The medical implant device 300 can comprise a light emitting diode (LED) configured to emit infrared light signals and a light emitting diode (LED) configured to emit red light signals. For example, the light source 370 can comprise a light emitting diode (LED) configured to emit light having a wavelength of about 940 nanometers (nm) and a light emitting diode (LED) configured to emit light having a wavelength of about 660 nanometer (nm). In some instances, the light sensor 380 can comprise one or more photodetectors, such as photodiodes, configured to detect the infrared light signals and red light signals.

It will be understood that more or fewer anchor components can be used. Any number of mechanical fasteners can be used to secure the anchor components to the heart wall. In some instances, one or more portions of the anchor components can be flexible, deformable and/or elastic to accommodate for any changes due to beating of the heart while maintaining the housing at a desired position over the extraluminal surface of the coronary sinus.

Although description of the medical implant device 300 herein refers primarily to the coronary sinus, it will be understood that one or more other coronary veins can be applicable. For example, the medical implant device 300 can be configured to provide light absorption information of blood flowing through another coronary vein of the greater cardiac venous system, such as the great cardiac vein. For example, the housing 304 receiving the light source 370 and the light sensor 380 can be configured to be positioned over an extraluminal surface of the great cardiac vein to measure light absorption information of blood flowing through the great cardiac vein. The first and second anchor components 330, 350 can facilitate securing the medical implant device 300 to respective heart wall portions adjacent to the great cardiac vein.

A medical implant device as described herein can be delivered using any number of techniques, including minimally invasive techniques. In some instances, a method for delivering the medical implant device can comprise any number of minimally invasive approaches for accessing the pericardial space. In some instances, a method for delivering the medical implant device can comprise a fluoro-guided minimally invasive delivery method. For example, a catheter placed in the coronary sinus can be used as a fluoroscopic target for a delivery system of the medical implant device. In some instances, a method for delivering the medical implant device can comprise using a magnetic component to guide the delivery. For example, a magnetic delivery guide component can be positioned within a coronary vein, including the coronary sinus or the great cardiac vein, to guide placement of at least a portion of a medical implant device over an extraluminal surface of the coronary vein, such as an extraluminal surface of the coronary sinus or great cardiac vein. The portion of the medical implant device positioned over the extraluminal surface can comprise another magnetic component configured to magnetically couple to the magnetic delivery guide component within the coronary vein, facilitating positioning of the portion of the medical implant device at a desired location over the extraluminal surface. A magnetic guide component within the coronary sinus or great cardiac vein can facilitate positioning of the medical implant device at a desire location over the extraluminal surface of the coronary sinus or great cardiac vein. As described in further detail herein, a magnetic anchor component of a medical implant device described herein can be used as a magnetic delivery guide for delivering the medical implant device to the desired location.

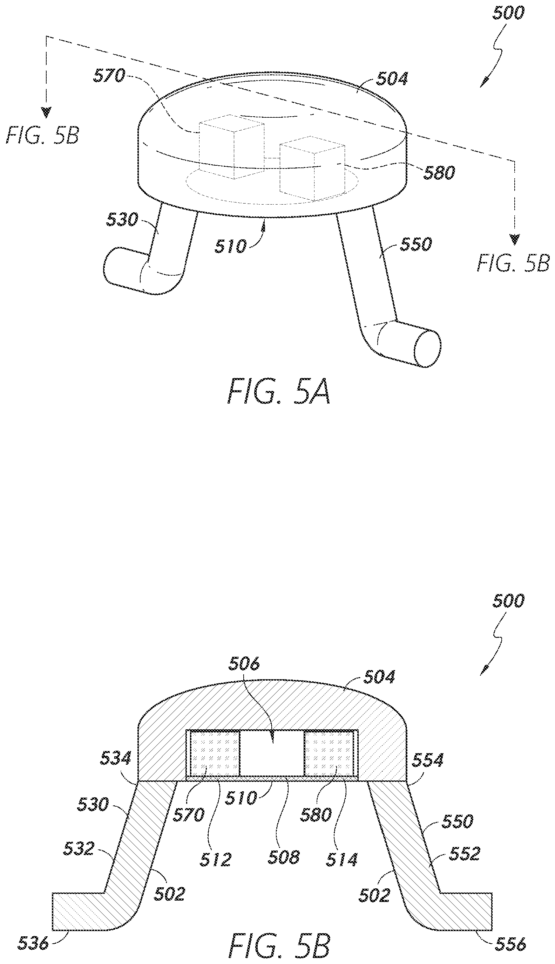

Figure 5A is a perspective view, and Figure 5B is a side cross-sectional view, of another example of a medical implant device 500 configured to be positioned over an extraluminal surface of a coronary sinus. The medical implant device 500 can comprise a housing 504 and an anchor 502 associated with a first wall portion 508 of the housing 504. A light source 570 and a light sensor 580 can be received within a cavity 506 of the housing 504. The anchor 502 can comprise a first anchor component 530 and a second anchor component 550 coupled to respective portions of the first wall portion 508. The first anchor component 530 can comprise a first anchor portion 532 oriented at an angle relative to the first wall portion 508, including a first surface 510 of the first wall portion 508. The second anchor component 550 can comprise a second anchor portion 552 oriented at an angle relative to the first wall portion 508, including the first surface 510 of the first wall portion 508. The first and second anchor components 530, 550 can extend between the first wall portion 508 and the heart wall such that the first and second anchor components 530, 550 can facilitate securing the medical implant device 500 to heart wall portions adjacent to the coronary sinus. The first wall portion 508 can comprise a first surface 510 configured to be oriented toward the extraluminal surface of the coronary sinus. The first surface 510 of the first wall portion 508 can be positioned over and/or in contact with the extraluminal surface of the coronary sinus. In some instances, the first and second anchor components 530, 550 can extend between the first surface 510 and respective portions of the heart wall on either side of the coronary sinus.

In some instances, the first and second anchor components 530, 550 can be coupled to respective portions of the first wall portion 508 that are laterally disposed from a first and second light-transparent regions 512, 514 on the first wall portion 508. A first end portion 534, 554 of each of the first and second anchor components 530, 550 can be coupled to respective portions of the first surface 510 laterally disposed from the first and second light-transparent regions 512, 514 so as not to interfere with emission of light signals by the light source 570 into the coronary sinus and detection by the light sensor 580 of reflected light signals from the coronary sinus. In some instances, the first and second anchor components 530, 550 can be coupled to opposing portions on the first surface 510. A second end portion 536, 556 of each of the first and second anchor components 530, 550 can be configured to be coupled to heart wall portions on a first side and a second side of the coronary sinus, respectively.

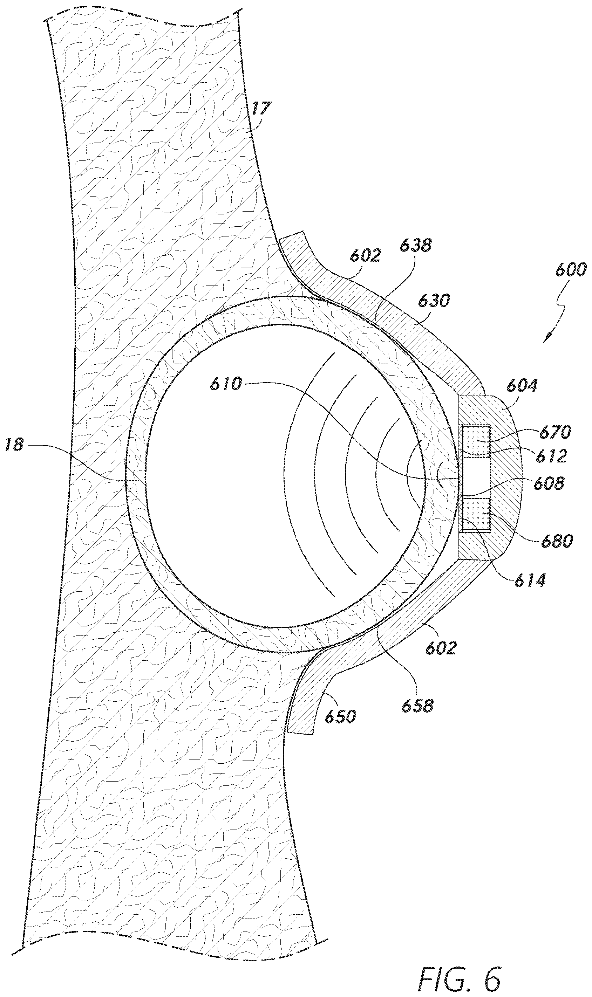

Figure 6 shows a side cross-sectional view of yet another example of a medical implant device 600 configured to be positioned over an extraluminal surface of a coronary sinus. The medical implant device 600 can comprise a housing 604 and an anchor 602 associated with the housing 604, where the anchor 602 includes at least a portion configured to be secured to a wall of a coronary sinus 18 to position the housing 604 over an extraluminal surface of the coronary sinus 18. The anchor 602 can comprise a first anchor component 630 and a second anchor component 650. At least a portion of each of the first anchor component 630 and the second anchor component 650 can extend between the housing 604 and a heart wall 17. Each of the first component 630 and the second anchor component 650, including respective portions extending between the housing 604 and the heart wall 17, can comprise a curved surface portion 638, 658 configured to be oriented toward the coronary sinus 18 while the housing 604 is positioned over the extraluminal surface of the coronary sinus 19. At least a portion of the curved surface portions 638, 658 can be secured to respective portions of the coronary sinus wall to facilitate positioning the housing 604 over the extraluminal surface of the coronary sinus 18. For example, a first wall portion 608 of the housing 604, including a first surface 610 of the first wall portion 608, can be positioned over and/or in contact with the extraluminal surface. The medical implant device 600 can comprise a light source 670 and a light sensor 680 received within a cavity 606 of the housing 604 and configured to provide measurements of light absorption properties of blood flowing through the coronary sinus 18. Light signals emitted by the light source 670 can pass through a first light-transparent region 612 on the first wall portion 608, and light signals reflected from the coronary sinus 18 can pass through a second light-transparent region 614 on the first wall portion 608. In some instances, the first and second light-transparent regions 612, 614 may not be distinct regions such that emitted light signals and reflected light signals can pass through the same light-transparent region on the first wall portion 608. For example, the first and second light-transparent regions 612, 614 can at least partially overlap. The first anchor component 630 and second anchor component 650 can be coupled to respective portions of the housing 604 without interfering with the emission and detection of light signals by the light source 670 and light sensor 680.

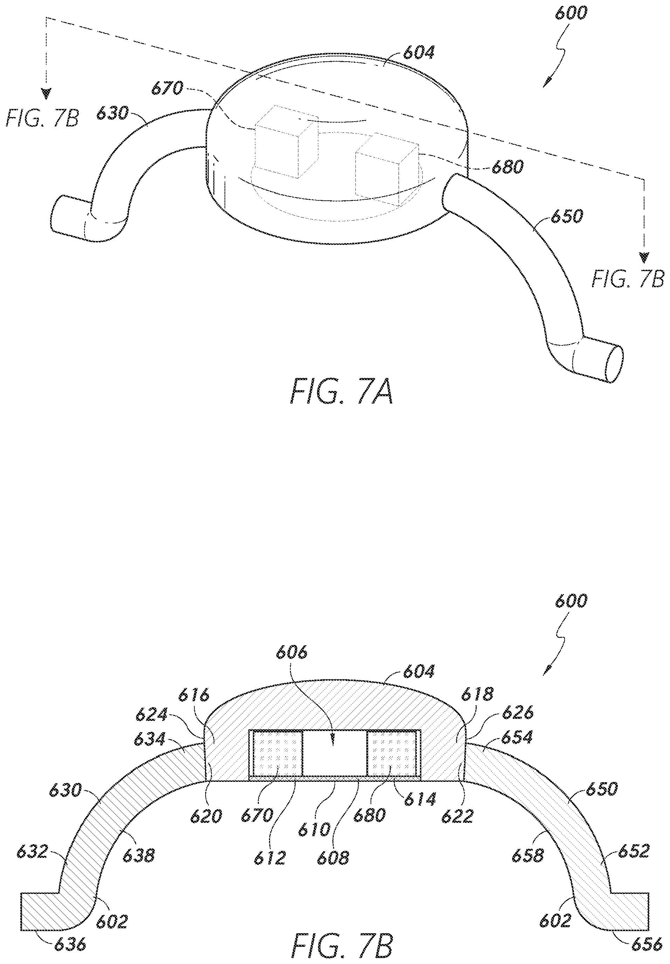

Figure 7A is a perspective view, and Figure 7B is a side cross-sectional view, of the medical implant device 600 described with reference to Figure 6. The first anchor component 630 can comprise a first anchor portion 632 and the second anchor component 650 can comprise a second anchor portion 652 extending between the housing 604 and a heart wall. Each of the first and second anchor portions 632, 652 can comprise a first and second curved surface portion 638, 658, respectively, configured to be oriented toward the coronary sinus. For example, the first curved surface portion 638 can be secured to a portion of the coronary sinus wall on a first side of the housing 604 and the second curved surface portion 658 can be secured to a portion of the coronary sinus wall on a second side of the housing 604. The first and second curved surface portions 638, 658 can be secured to the coronary sinus wall using any number of techniques, including for example using an adhesive. At least a portion of each of the curved surface portions 638, 658 can be shaped to facilitate conforming the respective portions to the coronary sinus wall so as to facilitate securing the respective portions thereto.

The first and second anchor components 630, 650 can be coupled to portions of the housing 604 such that the anchor components 630, 650 do not interfere with emission of light signals by the light source 670 into the coronary sinus and detection by the light sensor 680 of reflected light signals from the coronary sinus. The first anchor component 630 and second anchor component 650 can be coupled to a first housing portion 616 and a second housing portion 618, respectively, which are each laterally disposed from the first and second light-transparent regions 612, 614. A first end portion 634, 654 of each of the first and second anchor components 630, 650 can be coupled to the respective housing portions. In some instances, the first housing portion 616 can comprise a first lateral wall portion 620 and the second housing portion 618 can comprise a second lateral wall portion 622. For example, the first lateral wall portion 620 can comprise a first external lateral surface 624 to which the first anchor component 630 can be coupled. The second lateral wall portion 622 can comprise a second external lateral surface 626 to which the second anchor component 650 can be coupled. The first and second lateral housing portions 620, 622 can be at an angle relative to the first wall portion 608. In some instances, the first and second lateral housing portions 620, 622 can have an orientation perpendicular or substantially perpendicular to that of the first wall portion 608. In some instances, the first housing portion 616 and the second housing portion 618 can be at opposing positions on the housing 604. The first external surface 624 and the second external surface 626 can each have a perpendicular or substantially perpendicular orientation relative to the first wall portion 608, including the first surface 610 of the first wall portion 608. The first end portion 634 of the first anchor component 630 can be coupled to the first lateral wall portion 620, including the first external lateral surface 624. The first end portion 654 of the second anchor component 650 can be coupled to the second lateral wall portion 622, including the second external lateral surface 626.

In some instances, a second end portion 636, 656 of each of the first and second anchor components 630, 650 can be configured to be coupled to respective heart wall portions on a first side and a second side of the coronary sinus. Alternatively, the first and second anchor components 630, 650 may not be secured to the heart wall. For example, the first and second anchor components 630, 650, such as the first and second curved surface portions 638, 658, can be configured to be coupled to the coronary sinus wall, without being coupled to the heart wall.

The medical implant devices 500, 600 described with reference to Figures 5 through 7 can have one or more other features of the medical implant device 300 described with reference to Figures 3 and 4. For example, each of the first and second anchor components 530, 550, 630, 650 can be mechanically secured to the heart wall. In some instances, each of the second end portions 536, 556, 636, 656 can comprise at least one of a clip, corkscrew and barb. In some instances, each of the second end portions 536, 556, 636, 656 can comprise openings configured to facilitate stitching of the second end portions 536, 556, 636, 656 to the heart wall. For example, the openings can receive a stitch, suture, cord and/or other fastener that is configured to be secured to the heart wall. The medical implant devices 500, 600 can be applicable to another coronary vein, including another coronary vein of the greater cardiac venous system. For example, the housings 504, 604 receiving the respective light source 570, 670 and light sensor 580, 680 can be configured to be positioned over an extraluminal surface of the great cardiac vein. The first and second anchor components 530, 550 can facilitate securing the medical implant device 500 to respective heart wall portions adjacent to the great cardiac vein. The first and second anchor components 630, 650 can facilitate securing the medical implant device 600 to respective heart wall portions adjacent to the great cardiac vein and/or the great cardiac vein wall.

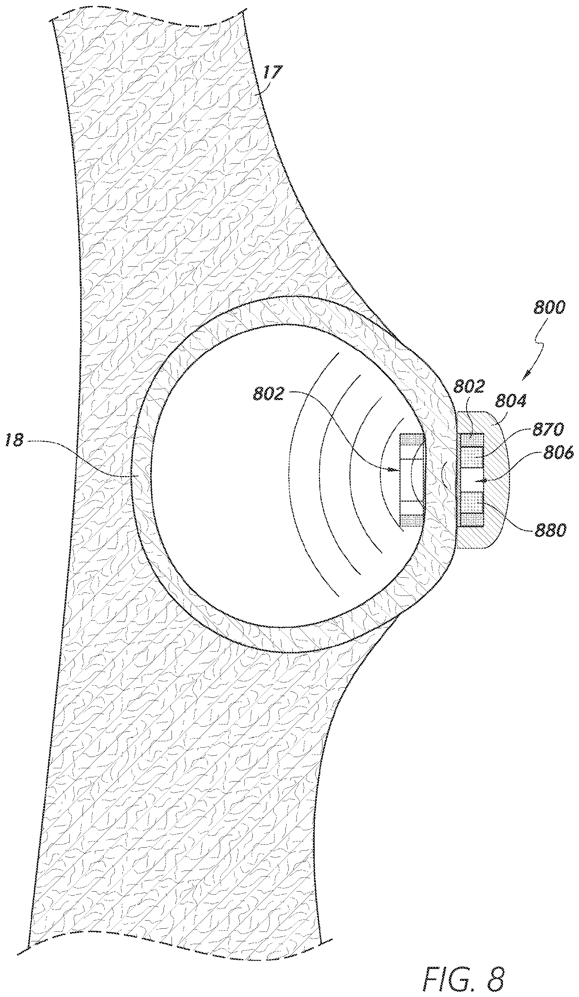

**In** some instances, a medical implant device can comprise an anchor configured to magnetically secure the medical implant device to the heart. Figure 8 is a side cross-sectional view of an example of a medical implant device 800 comprising a magnetic anchoring mechanism. The magnetic anchor mechanism can be configured to facilitate positioning the medical implant device 800 over an extraluminal surface of a coronary sinus. The medical implant device 800 can comprise a housing 804, and a light source 870 and a light sensor 880 received within a cavity 806 of the housing 804. An anchor 802 can be configured to magnetically secure the housing 804 over an extraluminal surface of a coronary sinus 18. The anchor 802 can comprise a first magnetic component 830 associated with the housing 804 positioned externally of the coronary sinus 18 and a second magnetic component 850 configured to be positioned within the coronary sinus 18. Magnetic coupling between the first and second magnetic components 830, 850 can secure the housing 804 over the extraluminal surface of the coronary sinus 18.

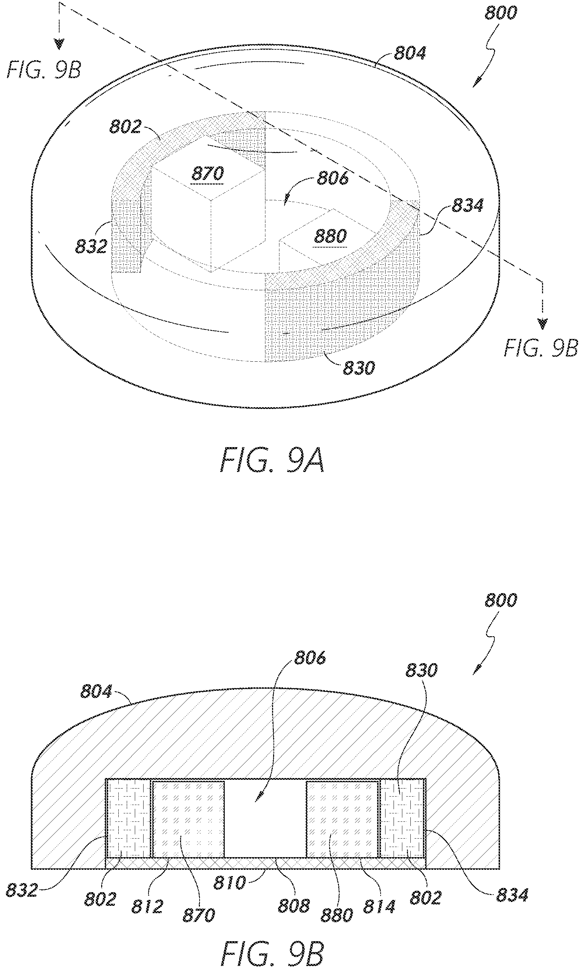

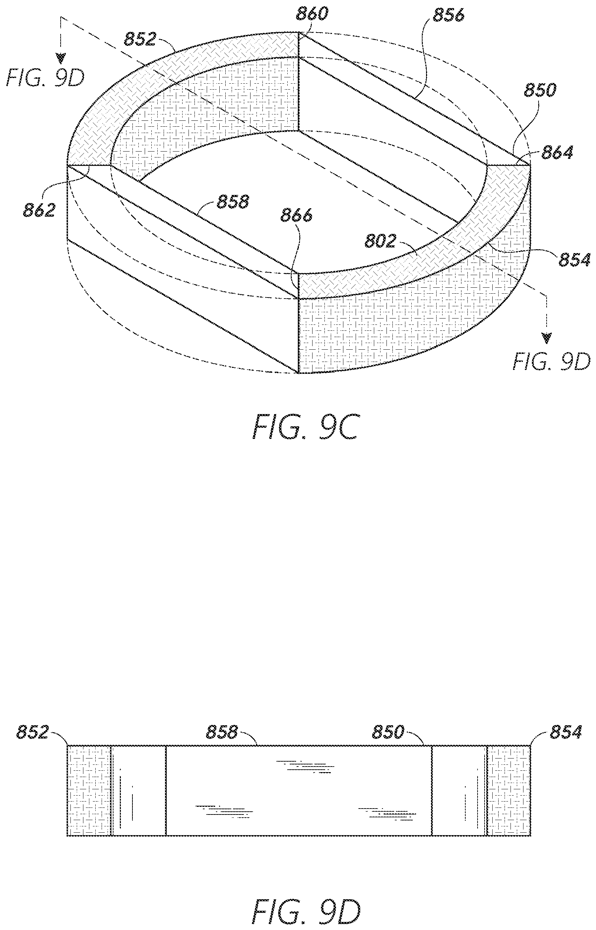

Figures 9A through 9D are various views of an example of the medical implant device 800 described with reference to Figure 8. Figure 9A is a perspective view, and Figure 9B is a side cross-sectional view, of the portion of the medical implant device 800 configured to be positioned over an extraluminal surface of a coronary sinus. Figure 9C is a perspective view, and Figure 9D is a side cross-sectional view, of the portion of the medical implant device 800 configured to be positioned within the coronary sinus, and magnetically coupled to the portion of the medical implant device 800 positioned externally of the coronary sinus. Referring to Figures 9A and 9B, a first wall portion 808 of the housing 804 can be configured to be positioned over and/or in contact with the extraluminal surface of the coronary sinus, including a first surface 810 of the first wall portion 808. The first wall portion 808 can comprise a first light-transparent region 812 to allow passage therethrough of emitted light signals from the light source 870 into the coronary sinus and a second light-transparent region 814 on the first wall portion 808 to allow passage therethrough of reflected light signals from the coronary sinus for detection by the light sensor 880. **In** some instances, the first and second light-transparent regions 812, 814 can at least partially overlap. For example, the first and second light-transparent regions 812, 814 may not be distinct regions such that emitted light signals and reflected light signals can pass through the same light-transparent region on the first wall portion 808. The first magnetic component 830 can be at a position within the housing 804 so as to not interfere with the transmission of light into the coronary sinus and detection of light reflected from the coronary sinus. For example, the first magnetic component 830 can be at one or more positions within the cavity 806 that are laterally disposed from the first and second light-transparent regions 812, 814.

In some instances, the first magnetic component 830 can be along at least a portion of a perimeter of the cavity 806 within the housing 804. In some instances, the first magnetic component 830 can comprise two or more discrete portions at respective positions within the cavity 806. For example, the first magnetic component 830 can comprise a first magnetic portion 832 and a second magnetic portion 834 each extending along a respective portion of the perimeter of the cavity 806 of the housing 804. In some instances, the first magnetic portion 832 and the second magnetic portion 834 can be at opposing positions around the perimeter of the cavity 806, for example, extending along opposing portions of the perimeter. The perimeter of the cavity 806 is shown in Figure 9A as comprising a rounded shape, such as a circular shape. For example, the first and second magnet portions 832, 834 can comprise a shape forming a segment of a circle. It will be understood that the perimeter of the cavity 806 can have a number of different shapes and the first and second magnetic portions 832, 834 can have shapes corresponding to the shape of the perimeter and/or portion of perimeter along which they extend.

Figures 9C and 9D show the second magnetic component 850 configured to be positioned within the coronary sinus. The second magnetic component 850 can comprise a configuration configured to mate with the first magnetic component 830. At least a portion of the second magnetic component 850 can be configured to be aligned with and magnetically coupled to the first magnetic component 830 through a coronary sinus wall portion so as to position the first light-transparent region 812 and the second light-transparent region 814 over and/or against the extraluminal surface of the coronary sinus. The second magnetic component 850 can comprise respective portions configured to magnetically couple with the first and second magnetic portions 832, 834 of the first magnetic component 830. For example, a first mating magnetic portion 852 and a second mating magnetic portion 854 of the second magnetic component 850 can be configured to magnetically couple to the first and second magnetic portions 832, 834, respectively.

**In** some instances, respective ends of the first and second mating magnetic portions 852, 854 can be coupled to one another. For example, the second magnetic component 850 can comprise a third portion 856 extending between first ends 860, 864 of the first and second mating magnetic portions 852, 854 and a fourth portion 858 extending between second ends 862, 866 of the first and second mating magnetic portions 852, 854. The first and second mating magnetic portions 852, 854 can be coupled to one another without interfering with the transmission of light into the coronary sinus and detection of light reflected from the coronary sinus. For example, the first and second mating magnetic portions 852, 854, and the third and fourth portions 856, 858 of the second magnetic component 850 can define an opening such that light signals can be transmitted therethrough. For example, the second magnetic component 850 can form a ring shape having a central opening configured to be aligned with the first and second light-transparent regions 812, 814 of the first wall portion 808.

Alternatively, the first magnetic component can extend circumferentially around the perimeter of the cavity of the housing, for example forming a ring shape. The second magnetic component can comprise a corresponding ring shape and configured to align with and magnetically couple to the circumferentially extending first magnetic component. It will be understood that a first and second magnetic components can comprise any number of configurations, including linear and/or curved portions. The first magnetic component can be at positions other than along the perimeter of the cavity, including positions which do not interfere with the delivery into and receipt of light signals from the coronary sinus.

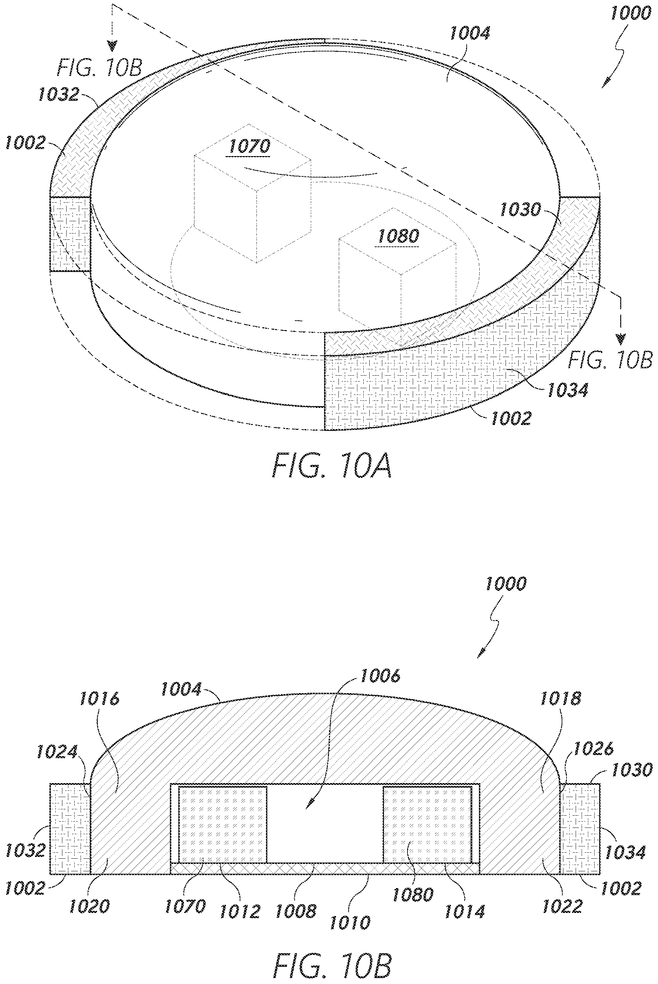

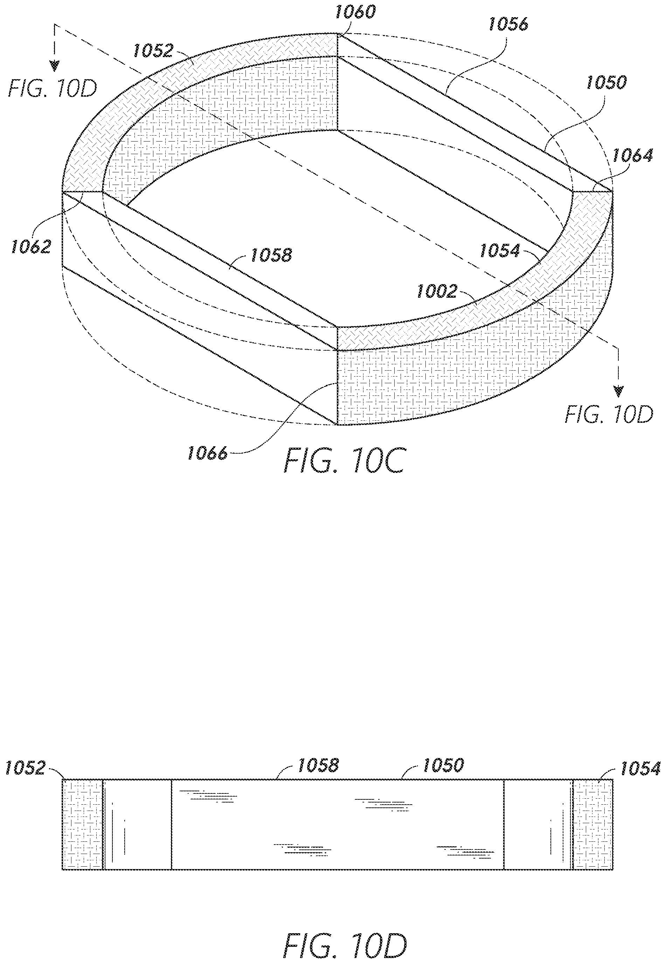

Figures 10A through 10D are various views of another example of a medical implant device 1000 comprising a magnetic anchoring mechanism. Figure 10A is a perspective view, and Figure 10B is a side cross-sectional view, of the portion of the medical implant device 1000 configured to be positioned over an extraluminal surface of a coronary sinus. Figure 10C is a perspective view, and Figure 10D is a side cross-sectional view, of the portion of the medical implant device 1000 configured to be positioned within the coronary sinus, and configured to be magnetically coupled to the portion of the medical implant device 1000 positioned externally of the coronary sinus.

The medical implant device 1000 can comprise a housing 1004 and an anchor 1002 configured to magnetically secure the housing 1004 over an extraluminal surface of a coronary sinus. A light source 1070 and a light sensor 1080 can be received within a cavity 1006 of the housing 1004. A first wall portion 1008 of the housing 1004 can be configured to be positioned over the extraluminal surface of the coronary sinus. For example, a first surface 1010 of the first wall portion 1008 can be over and in contact with the extraluminal surface of the coronary sinus. The first wall portion 1008 can comprise a first light-transparent region 1012 to allow passage therethrough of an emitted light signal from the light source 1070 and a second light-transparent region 1014 to allow passage therethrough of a reflected light signal from the coronary sinus for detection by the light sensor 1080. In some instances, the first and second light-transparent regions 1012, 1014 may not be distinct regions. For example, the first and second light-transparent regions 1012, 1014 can at least partially overlap. In some instances, emitted light signals and reflected light signals can pass through the same light-transparent region on the first wall portion 1008. The anchor 1002 can comprise a first magnetic component 1030 disposed externally of the housing 1004 and laterally disposed from the first and second light-transparent regions 1012, 1014. For example, the first magnetic component 1030 can comprise a first magnetic portion 1032 and a second magnetic portion 1034 coupled to a first and second housing portion 1016, 1018, respectively, that are laterally disposed from the first and second light-transparent regions 1012, 1014. In some instances, the first housing portion 1016 can comprise a first lateral wall portion 1020 and the second housing portion 1018 can comprise a second lateral wall portion 1022. In some instances, the first and second lateral housing portions 1020, 1022 can be at an angle relative to the first wall portion 1008. In some instances, the first and second lateral housing portions 1020, 1022 can have an orientation perpendicular or substantially perpendicular to that of the first wall portion 1008, including the first surface 1010. The first magnetic portion 1032 can be coupled a first external lateral surface 1024 of the first lateral wall portion 1020 and the second magnetic portion 1034 can be coupled to a second external lateral surface 1026 of the second lateral wall portion 1022. The first and second external lateral surfaces 1024, 1026 can have an orientation perpendicular or substantially perpendicular to that of the first wall portion 1008, including the first surface 1010. In some instances, the first and second lateral housing portions 1020, 1022 can be at opposing positions on the housing 1004.

In some instances, the first magnetic component 1030 can extend around at least a portion of an outer perimeter of the housing 1004. For example, the first magnetic portion 1032 and the second magnetic portion 1034 can each extend along a respective portion of the outer perimeter of the housing 1004. In some instances, the first magnetic portion 1032 and the second magnetic portion 1034 can extend along opposing portions of the outer perimeter.

Figures 10C and 10D show the second magnetic component 1050 configured to be positioned within the coronary sinus. The second magnetic component 1050 can comprise a configuration configured to mate with the first magnetic component 1030. For example, the second magnetic component 1050 can comprise respective portions configured to align and magnetically couple with portions of the first magnetic component 1030. A first mating magnetic portion 1052 and a second mating magnetic portion 1054 of the second magnetic component 1050 can be configured to mate with the first and second magnetic portions 1032, 1034 of the first magnetic component 1030, respectively.

The first and second mating magnetic portions 1052, 1054 can be coupled to one another without interfering with the transmission of light into the coronary sinus and detection of light reflected from the coronary sinus. In some instances, respective ends of the first and second mating magnetic portions 1052, 1054 can be coupled to one another. For example, the second magnetic component 1050 can comprise a third portion 1056 extending between first ends 1060, 1064 of the first and second mating magnetic portions 1052, 1054 and a fourth portion 1058 extending between second ends 1062, 1066 of the first and second mating magnetic portions 1052, 1054. The first and second mating magnetic portions 1052, 1054, and the third and fourth portions 1056, 1058 of the second magnetic component 1050 can form a ring shape having a central opening configured to be aligned with the first and second light-transparent regions 1012, 1014 of the first wall portion 1008 such that light signals can be transmitted therethrough.

In some instances, a first magnetic component can comprise more or fewer discrete portions at respective positions around the outer perimeter of the housing. Alternatively, the first magnetic component can extend circumferentially around the outer perimeter of the housing, for example forming a ring shape. The second magnetic component can comprise a corresponding ring shape and configured to align with and magnetically couple to the circumferentially extending first magnetic component. It will be understood that a first and second magnetic components can comprise any number of configurations, including linear and/or curved portions.

In some instances, the medical implant devices 800, 1000 can be applicable to one or more coronary veins other than the coronary sinus, including one or more other coronary veins of the greater cardiac venous system. For example, magnetic coupling between the first magnet components 830, 1030 and second magnetic components 850, 1050 can secure the respective housing 804, 1004 over the extraluminal surface of the great cardiac vein. The second magnetic components 850, 1050 can be positioned within the great cardiac vein.

In some instances, a second magnetic component of a medical implant device can be configured to be a magnetic delivery guide and used to guide the delivery of a housing of the medical implant device to a desired location over the extraluminal surface of coronary vein, including the coronary sinus or the great cardiac vein. For example, the second magnetic component can be advanced into the coronary sinus and positioned over an inner surface portion coronary sinus wall adjacent to the desired location. The housing comprising the first magnetic component can subsequently be positioned over the extraluminal surface. The first magnetic component can align with and magnetically couple with the second magnetic component within the coronary sinus. Alternatively, the second magnetic component can be advanced into the great cardiac vein and positioned over an inner surface portion great cardiac vein wall adjacent to the desired location. The housing comprising the first magnetic component can subsequently be positioned over the extraluminal surface of the great cardiac vein. The first magnetic component can align with and magnetically couple with the second magnetic component within the great cardiac vein.

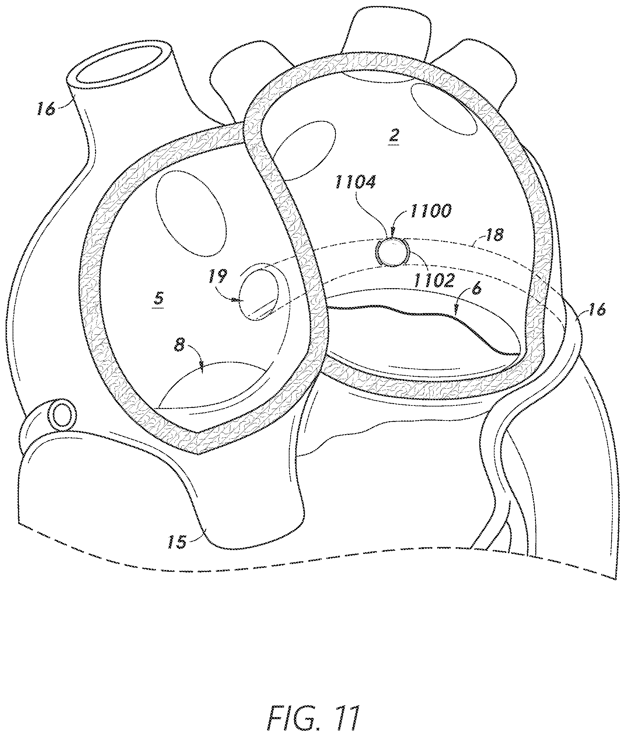

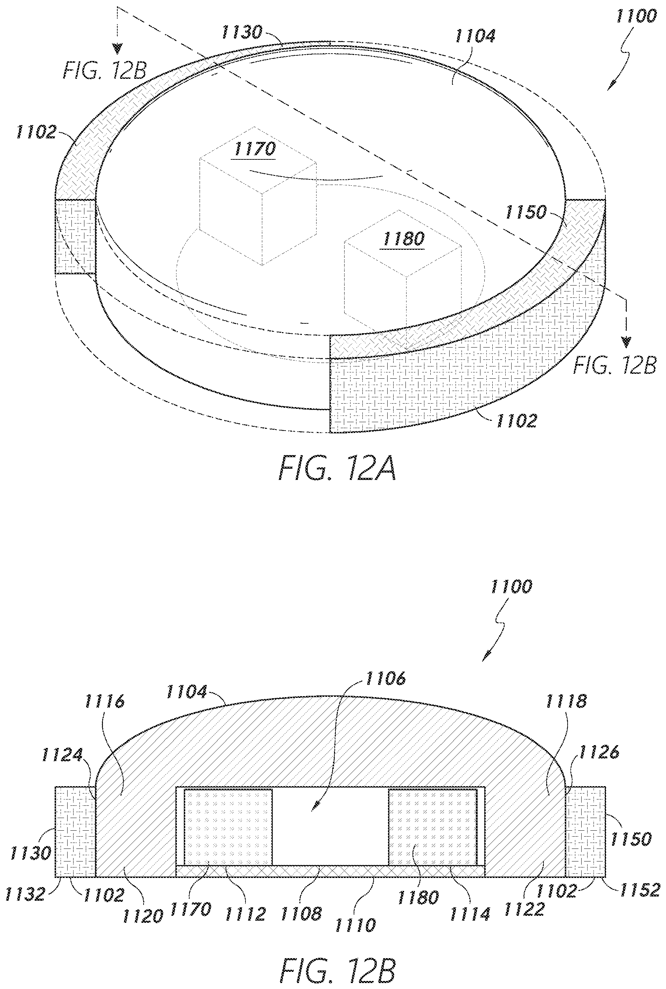

Figure 11 shows an example of a medical implant device 1100 positioned over an atrial wall in a left atrium 2. Figure 12A is a perspective view, and Figure 12B is side cross-sectional view, of the medical implant device 1100 described with reference to Figure 11. The medical implant device 1100 can be at least partially disposed within the left atrium 2. The medical implant device 1100 can comprise an anchor 1102 and a housing 1104 receiving a light source 1170 and a light sensor 1180 in a cavity 1106 of the housing 1104, where the anchor 1102 can be configured to secure the housing 1104 to the atrial wall. The housing 1104 can comprise a first wall portion 1108 configured to be positioned over the atrial wall. The first wall portion 1108 can comprise a first light-transparent region 1112 configured to allow passage therethrough of emitted light signals from the light source 1170 and a second light-transparent region 1114 configured to allow passage therethrough of reflected light signals from within the coronary sinus for detection by the light sensor 1180. In some instances, the first and second light-transparent regions 1112, 1114 may not be distinct regions. For example, the first and second light-transparent regions 1112, 1114 can at least partially overlap. In some instances, emitted light signals and reflected light signals can pass through the same light-transparent region on the first wall portion 1108. The anchor 1102 can be configured to be secured to the atrial wall such that the housing 1104, such as the first and second light-transparent regions 1112, 1114 of the first wall portion 1108, can be positioned over a portion of the wall of the left atrium 2 adjacent to a portion of a wall of a coronary sinus 18. For example, light signals emitted by the light source 1170 can pass through first light-transparent region 1112, and both the atrial wall and the coronary sinus wall to reach blood flowing through the coronary sinus 18. Reflected light from the coronary sinus 18 can pass through the coronary sinus wall, the atrial wall, and the second light-transparent region 1114 for detection by the light sensor 1180. In some instances, the anchor 1102 can comprise a first lateral anchor component 1130 and a second lateral anchor component 1150 associated with respective housing portions laterally disposed from the first light-transparent region 1112 and the second light-transparent region 1114 so as not to interfere with the transmission of light signals into and reflection of light signals from the coronary sinus.

Referring to Figures 12A and 12B, the first lateral anchor component 1130 and the second lateral anchor component 1150 can be associated with, such as coupled to, respective housing portions laterally disposed from the first light-transparent region 1112 and the second light-transparent region 1114. A first surface 1110 of the first wall portion 1108 can be oriented toward the atrial wall. In some instances, the first and second lateral anchor components 1130, 1150 can be secured to the atrial wall such that the first surface 1110 can be oriented toward and in contact with the left atrium facing surface of the atrial wall. Each of the first and second lateral anchor components 1130, 1150 can comprise a lateral portion 1132, 1152 having the same orientation as the first surface 1110 of the housing 1104. The first lateral portion 1132 of the first lateral anchor component 1130 and the second lateral portion 1152 of the second lateral anchor component 1150 can be configured to secure the medical implant device 1100 to the atrial wall. For example, the first and second lateral portions 1132, 1152 can be mechanically coupled a second atrial wall portion adjacent to the first atrial wall portion. In some instances, the first and second anchor components 1130, 1150 can comprise at least one of a clip, corkscrew and barb configured to be secured to the second atrial wall portion. In some instances, the first and second anchor components 1130, 1150 can comprise an opening configured to be stitched to the second atrial wall portion. For example, the openings can receive a stitch, suture, cord and/or other fastener that is configured to be secured to the atrial wall portion.

The first anchor component 1130 and second anchor component 1150 can be coupled to a first and second housing portion 1116, 1118, respectively, that are laterally disposed from the first and second light-transparent regions 1112, 1114. In some instances, the first housing portion 1116 can comprise a first lateral wall portion 1120 and the second housing portion 1118 can comprise a second lateral wall portion 1122. In some instances, the first and second lateral housing portions 1120, 1122 can be at an angle relative to the first wall portion 1108. In some instances, the first and second lateral housing portions 1120, 1122 can have an orientation perpendicular or substantially perpendicular to that of the first wall portion 1108, including the first surface 1110. The first anchor component 1130 can be coupled a first external lateral surface 1124 of the first lateral wall portion 1120 and the second anchor component 1150 can be coupled to a second external lateral surface 1126 of the second lateral wall portion 1122. In some instances, the first and second lateral housing portions 1120, 1122 can be at opposing positions on the housing 1104. The first and second external lateral surfaces 1124, 1126 can have an orientation perpendicular or substantially perpendicular to that of the first wall portion 1108, including the first surface 1110.

In some instances, the first and second anchor components 1130, 1150 can extend along respective portions of an outer perimeter of the housing 1104. In some instances, the first anchor component 1030 and the second anchor component 1150 can extend along opposing portions of the outer perimeter.

In some instances, an anchor can comprise more or fewer discrete portions at respective positions around the outer perimeter of the housing. Alternatively, the anchor can extend circumferentially around the entire outer perimeter of the housing, for example forming a ring shape. In some instances, a medical implant device configured to be positioned over an atrial wall can comprise one or more portions positioned over and/or in contact with a mitral valve annulus. The one or more portions of the medical implant device can be secured to the mitral valve annulus, including for example via an anchor comprising one or more features described herein. For example, the medical implant device can be mechanically secured to the mitral valve annulus using one or more of a clip, barb, corkscrew, and stitches.

In alternative instances, a medical implant device configured to be positioned over an atrial wall can comprise a magnetic anchoring mechanism. For example, the medical implant device can comprise one or more features of the medical implant devices 800, 1000 described with reference to Figures 8 through 10. A second magnetic component of the medical implant device can be positioned against an inner surface portion of the coronary sinus wall adjacent to the atrial wall. A first magnetic component coupled to and/or received by a housing of the medical implant device can be positioned over the desired portion of the atrial wall to align and magnetically couple to the second magnetic component in the coronary sinus.

In some instances, the medical implant device 1100 can be applicable to one or more coronary veins other than the coronary sinus, including one or more other coronary veins of the greater cardiac venous system. For example, the medical implant device 1100 may be positioned within a heart chamber for providing light absorption properties of blood flowing through the great cardiac vein. In some instances, the medical implant device 1100 can be positioned within the left atrium at a location to provide light absorption properties of blood flowing through the great cardiac vein. The anchor 1102 can be configured to be secured to the atrial wall such that the housing 1104, such as the first and second light-transparent regions 1112, 1114 of the first wall portion 1108, can be positioned over a portion of the wall of the left atrium adjacent to a portion of a wall of the great cardiac vein. In some instances, the medical implant device can comprise a magnetic anchoring mechanism. A second magnetic component of the medical implant device can be positioned against an inner surface portion of the great cardiac wall adjacent to the atrial wall. A first magnetic component coupled to and/or received by a housing of the medical implant device can be positioned over the desired portion of the atrial wall to align and magnetically couple to the second magnetic component in the great cardiac vein.

In some instances, a medical implant device can be positioned within another chamber of the heart to provide light absorption properties of blood flowing through the coronary sinus. In some instances, the medical implant device can be positioned within the left atrial appendage. The medical implant device can be secured within the left atrial appendage using a scaffolding component. In some instances, a light source and a light sensor of the medical implant device can emit light signals into the coronary sinus and receive reflected light signals from the coronary sinus through one or more heart wall portions adjacent to the coronary sinus.

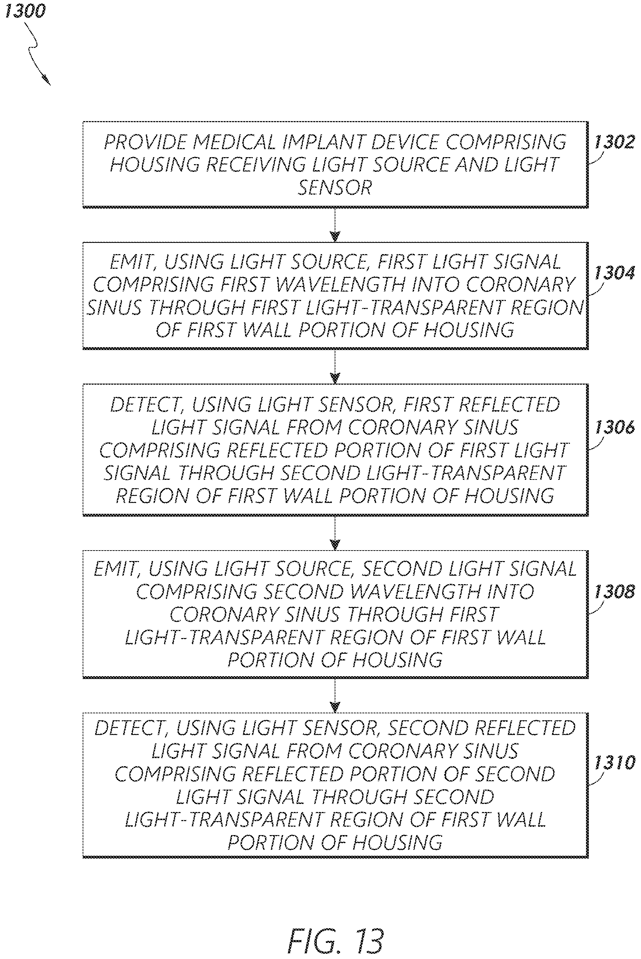

Figure 13 is a process flow diagram of an example of a process 1300 for providing measurements used to determine oxygen saturation level of blood flowing through the coronary sinus. **In** block 1302, the process can involve providing a medical implant device comprising one or more features described herein. For example, the medical implant device can comprise a housing receiving a light source and a light sensor. A first wall portion of the housing can comprise a first light-transparent region to allow passage therethrough of emitted light signals and a second light-transparent region to allow passage therethrough of reflected light signals. The light source can be configured to generate the emitted light signals. The emitted light signals from the light source can pass through the first light-transparent region for transmission into the coronary sinus. The reflected light signals can comprise portions of the emitted light signals not absorbed within the coronary sinus and reflected from the coronary sinus. The reflected light signals can pass through the second light-transparent region for detection by the light sensor.

In block 1304, the process can involve emitting, using the light source, a first light signal comprising a first wavelength into the coronary sinus through the first light-transparent region of the first wall portion. In block 1306, the process can involve detecting, using the light sensor, a first reflected light signal from the coronary sinus comprising a reflected portion of the first light signal through the second light-transparent region of the first wall portion. In block 1308, the process can involve emitting, using the light source, a second light signal comprising a second wavelength into a coronary sinus through the first light-transparent region of the first wall portion. In block 1310, the process can involve detecting, using the light sensor, a second reflected light signal from the coronary sinus comprising a reflected portion of the second light signal through the second light-transparent region of the first wall portion.

**In** some instances, the first wall portion of the medical implant device can be positioned over an extraluminal surface of the coronary sinus. A first surface of the first wall portion can be oriented toward and/or in contact with the extraluminal surface of the coronary sinus such that the emitted light signals from the light source can be transmitted through the first light-transparent region, and into the coronary sinus through the coronary sinus wall. Reflected light signals from the coronary sinus can pass through the coronary sinus wall and the second light-transparent region for detection by the light sensor. In some instances, emitting the first light signal and emitting the second light signal each comprise emitting a respective light signal through a coronary sinus wall portion. For example, a first light signal comprising an infrared light signal and/or a second light signal comprising a red light signal can travel from the light source through the first light-transparent region and the coronary sinus wall portion, and into the coronary sinus. Detecting the first reflected light signal and detecting the second reflected light signal can each comprise receiving a respective reflected light signal through the same and/or another coronary sinus wall portion.

In some instances, the first wall portion of the medical implant device can be positioned over an atrial wall portion adjacent to a coronary sinus wall portion. The first wall portion of the medical implant device can be configured to be positioned over a surface of a first atrial wall portion adjacent to a coronary sinus wall portion. The surface of the first atrial wall portion can be oriented toward a left atrium. For example, a first surface of the first wall portion can be over and/or in contact with a surface of the atrial wall portion oriented toward the left atrium such that light signals emitted from the light source can pass through the first light-transparent region, the atrial wall portion and the adjacent coronary sinus wall portion, and into the coronary sinus. The reflected light signals from the coronary sinus can pass through a coronary sinus wall portion, an adjacent portion of the atrial wall and the second light-transparent region for detection by the light sensor. In some instances, emitting the first light signal and emitting the second light signal can each comprise emitting a respective light signal through an atrial wall portion. Detecting the first reflected light signal and detecting the second reflected light signal can each comprise receiving a respective reflected light signal through the same and/or another atrial wall portion.

It will be understood that one or more processes described herein can be applicable to one or more coronary veins other than the coronary sinus, including one or more other coronary veins of the greater cardiac venous system. For example, the first wall portion of the medical implant device can be positioned over an extraluminal surface of a great cardiac vein to measure light absorption of blood flowing through the great cardiac vein. A first surface of the first wall portion can be oriented toward and/or in contact with the extraluminal surface of the great cardiac vein such that the emitted light signals from the light source can be transmitted through the first light-transparent region, through a portion of a great cardiac vein wall and into the great cardiac vein. Reflected light signals from the great cardiac vein can pass through the wall of the great cardiac vein and the second light-transparent region for detection by the light sensor. In some instances, emitting the first light signal and emitting the second light signal each comprise emitting a respective light signal through the great cardiac vein wall portion. For example, a first light signal comprising an infrared light signal and/or a second light signal comprising a red light signal can travel from the light source through the first light-transparent region and the great cardiac vein wall portion, and into the great cardiac vein. Detecting the first reflected light signal and detecting the second reflected light signal can each comprise receiving a respective reflected light signal through the same and/or another great cardiac vein wall portion.

In some instances, the first wall portion of the medical implant device can be positioned over a heart chamber wall portion, such as an atrial wall portion, adjacent to a great cardiac vein wall portion. The first wall portion of the medical implant device can be configured to be positioned over a surface of a first atrial wall portion adjacent to a great cardiac vein wall portion. The surface of the first atrial wall portion can be oriented toward a left atrium. For example, a first surface of the first wall portion can be over and/or in contact with a surface of the atrial wall portion oriented toward the left atrium such that light signals emitted from the light source can pass through the first light-transparent region, the atrial wall portion and the adjacent great cardiac vein wall portion, and into the great cardiac vein. The reflected light signals from the great cardiac vein can pass through a great cardiac vein wall portion, an adjacent portion of the atrial wall and the second light-transparent region for detection by the light sensor. In some instances, emitting the first light signal and emitting the second light signal can each comprise emitting a respective light signal through an atrial wall portion. Detecting the first reflected light signal and detecting the second reflected light signal can each comprise receiving a respective reflected light signal through the same and/or another atrial wall portion.

As described herein, oxygenated hemoglobin and deoxygenated hemoglobin can absorb infrared light and red light differently. Oxygenated hemoglobin can absorb more infrared light than red light. Deoxygenated hemoglobin can absorb more red light than infrared light. The difference in light absorption properties of hemoglobin can be used to determine blood oxygen saturation information for the blood flowing through a coronary vein, including the coronary sinus or the great cardiac vein. In some instances, the light source can be configured to emit a first light signal at a first wavelength in an infrared range. For example, the first light signal can have a wavelength of about 660 nanometer (nm). In some instances, the light source can be configured to emit a second light signal at a second wavelength in the red range. For example, the second light signal have a wavelength of about 940 nanometers (nm). The medical implant device can measure and/or detect the light absorption properties of blood flowing through the coronary vein, such as the coronary sinus or great cardiac vein. The light absorption properties can be used to determine oxygen saturation level of the blood.

Emission of light signal by the light source and detection of reflected light signal by the light sensor can occur at any number of different time intervals. In some instances, the light source and light sensor can be configured to continuously emit light and detect light for continuous monitoring of oxygen saturation for blood flowing through the coronary vein. In some instances, light source and light sensor can be configured to emit light and detect light at predetermined intervals, including for example, for a predetermined period every hour, day, and/or week. In some instances, the light source and light sensor can be triggered and/or activated by an external stimulus. The external stimulus can be generated by an operator and/or auto-generated at a preset time interval, such as for on-demand measurements. For example, the light source and light sensor can be triggered and/or activated by an external stimulus using any number of wireless communication techniques to allow an operator to initiate emission and detection of light signals, such as for on-demand measurements, and/or to set time intervals between and/or number of measurements. The light source and light sensor can be triggered and/or activated by an external stimulus using any number of wireless communication techniques to allow an operator to initiate emission and detection of light signals, such as for on-demand measurements, and/or to set time intervals between and/or the number of measurements. Activation and/or control of the light source and light sensor can be performed remotely by an operator at a location different from that of the patient, such as a different room, and/or building.

In some instances, the medical implant device can be configured to perform one or more other steps for determining the blood oxygen saturation level. In some instances, computation of blood oxygen saturation using the light absorption properties of the blood can be performed by the medical implant device. In some instances, one or more other steps for determining the blood oxygen saturation level can be performed externally from the medical implant device. For example, the light absorption properties of the blood determined by the light source and light sensor can be communicated to an external device, such as by wireless communication techniques, including a device external of the patient, to be used for calculating the blood oxygen saturation information.

Monitoring of oxygen utilization by the myocardium can facilitate detection and/or prediction of a number of conditions. In some instances, coronary vein, including coronary sinus and/or great cardiac vein, blood saturation can be used to predict acute decompensated heart failure (ADHF). For example, blood oxygen saturation information obtained using the light absorption properties can be combined with one or more other physiological metrics to determine a possible acute decompensated heart failure (ADHF) event, including for example, heart rate, blood pressure, and/or electrophysiology measurements (e.g., EKG). **In** some instances, a drop in blood oxygen saturation can be due to insufficient coronary blood perfusion, indicating for example, epicardial coronary stenosis and/or coronary microvascular disease. A decrease in blood oxygen saturation can also be due to demand ischemia, such as higher oxygen demand than available oxygen supply. A high oxygen demand relative to oxygen supply can indicate hypertrophic cardiomyopathy, and/or tachycardia. Increase in blood oxygen saturation can indicate, for example, energy metabolism impairment in heart failure with preserved ejection fraction (HFpEF), myocardial fibrosis, scar formation and/or infiltrative disorders.

In some instances, a measurement of a systemic arterial blood oxygen saturation can be used in combination with a measurement of a coronary vein, such as coronary sinus and/or great cardiac vein, blood oxygen saturation. For example, one or more measurements of the systemic arterial blood oxygen saturation can be used in combination with one or more coronary vein blood oxygen saturation measurements to facilitate determining the health of the heart, including prediction of acute decompensated heart failure (ADHF). In some instances, one or more systemic arterial blood oxygen saturation measurements can provide a blood oxygen saturation baseline for a patient, for example serving as a reference point against which coronary vein blood oxygen saturation measurements can be compared in determining whether coronary vein blood oxygen saturation measurements indicate a problem with heart function or an oxygenation problem elsewhere in the body (*e.g.,* a deterioration of blood oxygenation in the lungs). In some instances, a difference between the systemic arterial blood oxygen saturation and coronary vein blood oxygen saturation can be used. A change in the difference between the systemic arterial blood oxygen saturation and the coronary vein blood oxygen saturation can be used to diagnose a problem with heart function. For example, a change in the difference between the systemic arterial blood oxygen saturation and the coronary vein blood oxygen saturation in combination with a change in the coronary vein blood oxygen saturation can indicate a problem with the heart. A change in the difference between the systemic arterial blood oxygen saturation and the coronary vein blood oxygen saturation in combination with a change in the systemic arterial blood oxygen saturation can indicate a problem outside of the heart. The systemic blood oxygen saturation measurements and coronary vein blood oxygen saturation measurements may or may not be taken simultaneously. In some instances, the systemic blood oxygen saturation measurements and coronary vein blood oxygen saturation measurements can be taken close enough in time to allow desired comparison between the two, including within an hour, a day and/or a week apart.

In some instances, coronary vein, including coronary sinus and/or great cardiac vein, blood oxygen saturation can be used in combination with other physiological metrics to monitor the physiological state of the patient, such as to improve tracking of disease progress and/or diagnosis. In some instances, coronary vein blood oxygen saturation can be used in combination with hemodynamic metrics (e.g., intracardiac pressures, systemic blood pressure, heart rate), electrophysiology measurements (*e.g.,* EKG), systemic oxygen saturation (*e.g.,* systemic SaO2), venous oxygen saturation (SvO2), and/or indices of aerobic and anaerobic metabolism (e.g., pH, lactate). Coronary vein blood oxygen saturation can be used in combination with other physiological metrics to track progress and/or facilitate diagnosis of various cardiovascular diseases and/or abnormalities.

The above method(s) can be performed on a living animal or on a simulation, such as on a cadaver, cadaver heart, anthropomorphic ghost, simulator (e.g., with body parts, heart, tissue, etc. being simulated).

Depending on the embodiment, certain acts, events, or functions of any of the processes or algorithms described herein can be performed in a different sequence, may be added, merged, or left out altogether. Thus, in certain examples, not all described acts or events are necessary for the practice of the processes.

Conditional language used herein, such as, among others, "can," "could," "might," "may," "e.g.," and the like, unless specifically stated otherwise, or otherwise understood within the context as used, is intended in its ordinary sense and is generally intended to convey that certain examples include, while other examples do not include, certain features, elements and/or steps. Thus, such conditional language is not generally intended to imply that features, elements and/or steps are in any way required for one or more examples or that one or more examples necessarily include logic for deciding, with or without author input or prompting, whether these features, elements and/or steps are included or are to be performed in any particular embodiment. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain examples require at least one of X, at least one of Y and at least one of Z to each be present.

It should be appreciated that in the above description of examples, various features are sometimes grouped together in a single embodiment, Figure, or description thereof for the purpose of streamlining the disclosure and aiding in the understanding of one or more of the various aspects. This method of disclosure, however, is not to be interpreted as reflecting an intention that any claim require more features than are expressly recited in that claim. Moreover, any components, features, or steps illustrated and/or described in a particular example herein can be applied to or used with any other example(s). Further, no component, feature, step, or group of components, features, or steps are necessary or indispensable for each embodiment. Thus, it is intended that the scope of the claimed inventions should not be limited by the particular examples described above, but should be determined only by a fair reading of the appended claims.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). **In** addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which the examples belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

## Claims

1. A medical implant device (300), comprising:
a light source (370) and a light sensor (380); and
a housing (304) configured to receive the light source and the light sensor, the housing comprising:
a first wall portion (308) configured to be positioned over an extraluminal surface of a coronary sinus, the first wall portion comprising a first surface (310) oriented toward the extraluminal surface,
a first light-transparent region (312) of the first wall portion configured to allow passage therethrough of an emitted light signal from the light source into the coronary sinus, and
a second light-transparent region (314) of the first wall portion configured to allow passage therethrough of a reflected light signal from within the coronary sinus for detection by the light sensor.

2. The device of claim 1, further comprising an anchor (302) configured to mechanically couple to a heart wall portion adjacent to the coronary sinus and hold the first wall portion over the extraluminal surface of the coronary sinus.

3. The device of claim 2, wherein the anchor (302) comprises a portion oriented at an angle relative to the first surface (310) of the housing and configured to extend between the housing and the heart wall portion to allow securing the housing to the heart wall portion.

4. The device of claim 3, wherein the portion of the anchor comprises a curved surface portion (638, 658) configured to be oriented toward and secured to a portion of the extraluminal surface.

5. The device of claim 3 or 4, wherein:
the portion of the anchor comprises a first anchor portion (332) coupled to a first housing portion (316) laterally disposed from the first and second light-transparent regions and oriented at an angle relative to the first surface, and configured to be secured to a first heart wall portion on a first side of the coronary sinus; and
the anchor comprises a second anchor portion (352) coupled to a second housing portion (318) laterally disposed from the first and second light-transparent regions and oriented at an angle relative to the first surface, and configured to be secured to a second heart wall portion on a second side of the coronary sinus.

6. The device of claim 5, wherein the first housing portion (316) and the second housing portion (318) are at opposing positions.

7. The device of claim 5, wherein the first anchor portion (332) and second anchor portion (352) each comprise at least one of a clip, corkscrew, barb and respective openings configure to receive a stitch, configured to be secured to the heart wall portion.

8. The device of claim 2, wherein the anchor comprises:
a first lateral anchor portion (334) coupled to a first housing portion (320) laterally disposed from the first and second light-transparent regions; and
a second lateral anchor portion (354) coupled to a second housing portion (322) laterally disposed from the first and second light-transparent regions,
each of the first lateral anchor portion and the second lateral anchor portion comprising the same orientation as the first surface (310) and the first housing portion and the second housing portion are opposing portions of the housing.

9. The device of claim 1, further comprising:
a first magnetic component (830), wherein the housing is associated with the first magnetic component; and
a second magnetic component (850) configured to be positioned within the coronary sinus to be aligned with and magnetically couple to the first magnetic component through a coronary sinus wall portion to position the first light-transparent region and the second light-transparent region over the extraluminal surface of the coronary sinus.

10. The device of claim 9, wherein:
the first magnetic component (1030) comprises a portion received within the housing at a position laterally disposed from the first and second light-transparent regions; and/or
the first magnetic component (1030) extends circumferentially around an outer perimeter of the housing (1004) and wherein the second magnetic component (1050) comprises a ring shape configured to be aligned with the first magnetic component; and/or
wherein the first magnetic component (1030) comprises a first magnetic portion (1032) coupled to a first lateral housing portion (1020) at a position laterally disposed from the first and second light-transparent regions.

11. A medical implant device, comprising:
a light source (1170) and a light sensor (1180); and
a housing (1104) configured to receive the light source and the light sensor, the housing comprising:
a first wall portion (1108) configured to be positioned over a surface of a first atrial wall portion adjacent to a coronary sinus wall portion, the surface of the atrial wall portion being oriented toward a left atrium, and the first wall portion comprising a first surface (1110) oriented toward the surface of the atrial wall portion,
a first light-transparent region (1112) on the first wall portion to allow passage therethrough of an emitted light signal from the light source, and
a second light-transparent region (1114) on the first wall portion to allow passage therethrough of a reflected light signal from the coronary sinus for detection by the light sensor.

12. The device of claim 11, further comprising an anchor (1102) associated with a housing portion laterally disposed from the first light-transparent region and the second light-transparent region, and comprising a lateral portion (1132, 1152) having the same orientation as the first surface of the housing, the lateral portion being configured to mechanically couple to a second atrial wall portion adjacent to the first atrial wall portion.

13. The device of claim 12, wherein the anchor (1102) comprises at least one of a clip, corkscrew, barb and an opening configured receive a stitch, configured to be secured to the second atrial wall portion.

14. The device claim 12 or 13, wherein:
the lateral portion comprises a first lateral anchor portion (1132) coupled to a first housing portion (1116) laterally disposed from the first and second light-transparent regions and comprising the same orientation as the first surface of the housing, and configured to be secured to a second atrial wall portion; and
the anchor comprises a second lateral anchor portion (1152) coupled to a second housing portion (1118) laterally disposed from the first and second light-transparent regions and comprising the same orientation as the first surface of the housing, and configured to be secured to a third atrial wall portion,
the first housing portion (1116) and the second housing portion (1118) are at opposing positions.

15. The device of claim 12 or 13, wherein the anchor (1102) extends circumferentially around the housing.

## Patentansprüche

1. Medizinische Implantat-Vorrichtung (300), umfassend:
eine Lichtquelle (370) und einen Lichtsensor (380); und
ein Gehäuse (304), das dazu konfiguriert ist, die Lichtquelle und den Lichtsensor aufzunehmen, wobei das Gehäuse umfasst:
einen ersten Wandabschnitt (308), der dazu konfiguriert ist, über einer extraluminalen Oberfläche eines Sinus Coronarius angeordnet zu werden, wobei der erste Wandabschnitt eine erste Oberfläche (310) aufweist, die in Richtung zu der extraluminalen Oberfläche ausgerichtet ist,
einen ersten lichtdurchlässigen Bereich (312) des ersten Wandabschnitts, der dazu konfiguriert ist, das Durchtreten eines von der Lichtquelle ausgesandten Lichtsignals in den Sinus Coronarius zu ermöglichen, und
einen zweiten lichtdurchlässigen Bereich (314) des ersten Wandabschnitts, der dazu konfiguriert ist, das Durchtreten eines von innerhalb des Sinus Coronarius reflektierten Lichtsignals zur Detektion durch den Lichtsensor zu ermöglichen.

2. Vorrichtung gemäß Anspruch 1, ferner einen Anker (302) umfassend, der dazu konfiguriert ist, mechanisch mit einem zu dem Sinus Coronarius benachbarten Abschnitt der Herzwand verbunden zu werden und den ersten Wandabschnitt über der extraluminalen Oberfläche des Sinus Coronarius zu halten.

3. Vorrichtung gemäß Anspruch 2, wobei der Anker (302) einen Abschnitt umfasst, der relativ zu der ersten Oberfläche (310) des Gehäuses in einem Winkel ausgerichtet ist und dazu konfiguriert ist, sich zwischen dem Gehäuse und dem Herzwandabschnitt zu erstrecken, um das Befestigen des Gehäuses an dem Herzwandabschnitt zu ermöglichen.

4. Vorrichtung gemäß Anspruch 3, wobei der Abschnitt des Ankers einen gekrümmten Oberflächenabschnitt (638, 658) umfasst, der dazu konfiguriert ist, in Richtung zu der extraluminalen Oberfläche orientiert und an einem Abschnitt derselben befestigt zu werden.

5. Vorrichtung gemäß Anspruch 3 oder 4, wobei:
der Abschnitt des Ankers einen ersten Ankerabschnitt (332) umfasst, der mit einem ersten Gehäuseabschnitt (316) verbunden ist, der seitlich zu dem ersten und dem zweiten lichtdurchlässigen Bereich angeordnet und in einem Winkel relativ zu der ersten Oberfläche orientiert ist, und der dazu konfiguriert ist, an einem ersten Herzwandabschnitt auf einer ersten Seite des Sinus Coronarius befestigt zu werden; und
der Anker einen zweiten Ankerabschnitt (352) umfasst, der mit einem zweiten Gehäuseabschnitt (318) verbunden ist, der seitlich zu dem ersten und dem zweiten lichtdurchlässigen Bereich angeordnet ist und relativ zu der ersten Oberfläche in einem Winkel orientiert ist, und der dazu konfiguriert ist, an einem zweiten Herzwandabschnitt auf einer zweiten Seite des Sinus Coronarius befestigt zu werden.

6. Vorrichtung gemäß Anspruch 5, wobei der erste Gehäuseabschnitt (316) und der zweite Gehäuseabschnitt (318) an entgegengesetzten Positionen angeordnet sind.

7. Vorrichtung gemäß Anspruch 5, wobei der erste Ankerabschnitt (332) und der zweite Ankerabschnitt (352) jeweils mindestens eines aufweisen von einem Clip, einem Korkenzieher, einem Dorn und entsprechende Öffnungen, die dazu konfiguriert sind, einen Nahtstich aufzunehmen, der dazu konfiguriert ist, an dem Herzwandabschnitt befestigt zu werden.

8. Vorrichtung gemäß Anspruch 2, wobei der Anker umfasst:
einen ersten lateralen Ankerabschnitt (334), der mit einem ersten Gehäuseabschnitt (320) verbunden ist, der seitlich zu dem ersten und dem zweiten lichtdurchlässigen Bereich angeordnet ist; und
einen zweiten lateralen Ankerabschnitt (354), der mit einem zweiten Gehäuseabschnitt (322) verbunden ist, der seitlich zu dem ersten und dem zweiten lichtdurchlässigen Bereich angeordnet ist,
wobei jeder von dem ersten lateralen Ankerabschnitt und dem zweiten lateralen Ankerabschnitt die gleiche Ausrichtung wie die erste Oberfläche (310) umfasst und der erste Gehäuseabschnitt und der zweite Gehäuseabschnitt entgegengesetzte Abschnitte des Gehäuses sind.

9. Vorrichtung gemäß Anspruch 1, darüber hinaus umfassend:
eine erste magnetische Komponente (830), wobei das Gehäuse der ersten magnetischen Komponente zugeordnet ist; und
eine zweite magnetische Komponente (850), die dazu konfiguriert ist, innerhalb des Sinus Coronarius positioniert zu werden, um mit der ersten magnetischen Komponente durch einen Wandabschnitt des Sinus Coronarius ausgerichtet und magnetisch gekoppelt zu werden, um den ersten lichtdurchlässigen Bereich und den zweiten lichtdurchlässigen Bereich über der extraluminalen Oberfläche des Sinus Coronarius anzuordnen.

10. Vorrichtung gemäß Anspruch 9, wobei:
die erste magnetische Komponente (1030) einen Abschnitt umfasst, der innerhalb des Gehäuses an einer Position aufgenommen ist, die seitlich zu dem ersten und dem zweiten lichtdurchlässigen Bereich angeordnet ist; und/oder
die erste magnetische Komponente (1030) sich umlaufend um einen Außenumfang des Gehäuses (1004) erstreckt, und wobei die zweite magnetische Komponente (1050) eine Ringform umfasst, die dazu konfiguriert ist, auf die ersten magnetischen Komponente ausgerichtet zu werden; und/oder
wobei die erste magnetische Komponente (1030) einen ersten magnetischen Abschnitt umfasst, der mit einem ersten seitlichen Gehäuseabschnitt (1020) verbunden ist, an einer Position, die seitlich zu dem ersten und dem zweiten lichtdurchlässigen Bereich angeordnet ist.

11. Medizinische Implantat-Vorrichtung, umfassend:
eine Lichtquelle (1170) und ein Lichtsensor (1180); und
ein Gehäuse (1104), das dazu konfiguriert ist, die Lichtquelle und den Lichtsensor aufzunehmen, wobei das Gehäuse umfasst:
einen ersten Wandabschnitt (1108), der dazu konfiguriert ist, über einer Oberfläche eines ersten Vorhofwandabschnitts benachbart zu einem Wandabschnitt des Sinus Coronarius angeordnet zu werden, wobei die Oberfläche des Vorhofwandabschnitts in Richtung zu dem linken Vorhof ausgerichtet ist, und wobei der erste Wandabschnitt eine erste Oberfläche (1110) umfasst, die in Richtung zu der Oberfläche des Vorhofwandabschnitts ausgerichtet ist,
einen ersten lichtdurchlässigen Bereich (1112) an dem ersten Wandabschnitt, um ein Durchtreten eines von der Lichtquelle ausgesandten Lichtsignals zu ermöglichen, und
einen zweiten lichtdurchlässigen Bereich (1114) an dem ersten Wandabschnitt, um ein Durchtreten eines vom Sinus Coronarius reflektierten Lichtsignals zur Detektion durch den Lichtsensor zu ermöglichen.

12. Vorrichtung gemäß Anspruch 11, ferner einen Anker (1102) umfassend, der mit einem Gehäuseabschnitt verbunden ist, der seitlich zu dem ersten lichtdurchlässigen Bereich und dem zweiten lichtdurchlässigen Bereich angeordnet ist, und einen Lateralabschnitt (1132, 1152) umfassend, der die gleiche Ausrichtung wie die erste Oberfläche des Gehäuses aufweist, wobei der Lateralabschnitt dazu konfiguriert ist, mechanisch mit einem zweiten Vorhofwandabschnitt verbunden zu werden, der benachbart zu dem ersten Vorhofwandabschnitt ist.

13. Vorrichtung gemäß Anspruch 12, wobei der Anker (1102) mindestens eines aufweist von einem Clip, einem Korkenzieher, einem Dorn und einer Öffnung, die dazu konfiguriert ist, einen Nahtstich aufzunehmen, der dazu konfiguriert ist, an dem zweiten Vorhofwandabschnitt befestigt zu werden.

14. Vorrichtung gemäß Anspruch 12 oder 13, wobei:
der laterale Abschnitt einen ersten lateralen Ankerabschnitt (1132) umfasst, der mit einem ersten Gehäuseabschnitt (1116) verbunden ist, der seitlich zu dem ersten und dem zweiten lichtdurchlässigen Bereich angeordnet ist und die gleiche Ausrichtung wie die erste Oberfläche des Gehäuses umfasst, und dazu konfiguriert ist, an einem zweiten Vorhofwandabschnitt befestigt zu werden; und
der Anker einen zweiten lateralen Ankerabschnitt (1152) umfasst, der mit einem zweiten Gehäuseabschnitt (1118) verbunden ist, der seitlich zu dem ersten lichtdurchlässigen Bereich und dem zweiten lichtdurchlässigen Bereich angeordnet ist und die gleiche Ausrichtung wie die erste Oberfläche des Gehäuses aufweist, und dazu konfiguriert ist, an einem dritten Vorhofwandabschnitt befestigt zu werden,
der erste Gehäuseabschnitt (1116) und der zweite Gehäuseabschnitt (1118) an gegenüberliegenden Positionen angeordnet sind.

15. Vorrichtung gemäß Anspruch 12 oder 13, wobei der Anker (1102) sich in Umfangsrichtung um das Gehäuse erstreckt.

## Revendications

1. Dispositif d'implant médical (300), comprenant :
une source de lumière (370) et un capteur de lumière (380) ; et
un boîtier (304) conçu pour recevoir la source de lumière et le capteur de lumière, le boîtier comprenant :
une première partie (308) de paroi conçue pour être positionnée sur une surface extraluminale d'un sinus coronaire, la première partie de paroi comprenant une première surface (310) orientée vers la surface extraluminale,
une première région transparente à la lumière (312) de la première partie de paroi conçue pour permettre le passage à travers celle-ci d'un signal de lumière émis depuis la source de lumière dans le sinus coronaire et
une seconde région transparente à la lumière (314) de la première partie de paroi conçue pour permettre le passage à travers celle-ci d'un signal de lumière réfléchie depuis l'intérieur du sinus coronaire pour une détection par le capteur de lumière.

2. Dispositif selon la revendication 1, comprenant en outre un ancrage (302) conçu pour s'accoupler mécaniquement à une partie de paroi cardiaque adjacente au sinus coronaire et pour maintenir la première partie de paroi sur la surface extraluminale du sinus coronaire.

3. Dispositif selon la revendication 2, l'ancrage (302) comprenant une partie orientée selon un angle par rapport à la première surface (310) du boîtier et conçue pour s'étendre entre le boîtier et la partie de paroi cardiaque afin de permettre la fixation du boîtier à la partie de paroi cardiaque.

4. Dispositif selon la revendication 3, la partie de l'ancrage comprenant une partie de surface incurvée (638, 658) conçue pour être orientée vers une partie de la surface extraluminale et fixée à celle-ci.

5. Dispositif selon la revendication 3 ou 4 :
la partie de l'ancrage comprenant une première partie (332) d'ancrage accouplée à une première partie (316) de boîtier disposée latéralement par rapport aux première et seconde régions transparentes à la lumière et orientée selon un angle par rapport à la première surface et conçue pour être fixée à une première partie de paroi cardiaque sur un premier côté du sinus coronaire ; et
l'ancrage comprenant une seconde partie (352) d'ancrage accouplée à une seconde partie (318) de boîtier disposée latéralement par rapport aux première et seconde régions transparentes à la lumière et orientée selon un angle par rapport à la première surface et conçue pour être fixée à une deuxième partie de paroi cardiaque sur un second côté du sinus coronaire.

6. Dispositif selon la revendication 5, la première partie (316) de boîtier et la seconde partie (318) de boîtier étant en des positions opposées.

7. Dispositif selon la revendication 5, la première partie (332) d'ancrage et la seconde partie (352) d'ancrage comprenant chacune au moins un élément parmi une pince, un tire-bouchon, une barbe et des ouvertures respectives conçues pour recevoir un point de couture, conçu pour être fixé à la partie de paroi cardiaque.

8. Dispositif selon la revendication 2, l'ancrage comprenant :
une première partie latérale (334) d'ancrage accouplée à une première partie (320) de boîtier disposée latéralement par rapport aux première et seconde régions transparentes à la lumière ; et
une seconde partie latérale (354) d'ancrage accouplée à une seconde partie (322) de boîtier disposée latéralement par rapport aux première et seconde régions transparentes à la lumière,
chaque partie parmi la première partie latérale d'ancrage et la seconde partie latérale d'ancrage comprenant la même orientation que celle de la première surface (310) et la première partie de boîtier et la seconde partie de boîtier étant des parties opposées du boîtier.

9. Dispositif selon la revendication 1, comprenant en outre :
un premier composant magnétique (830), le boîtier étant associé au premier composant magnétique ; et
un second composant magnétique (850) conçu pour être positionné à l'intérieur du sinus coronaire afin d'être aligné avec le premier composant magnétique et couplé magnétiquement à celui-ci par l'intermédiaire d'une partie de paroi de sinus coronaire en vue de positionner la première région transparente à la lumière et la seconde région transparente à la lumière sur la surface extraluminale du sinus coronaire.

10. Dispositif selon la revendication 9 :
le premier composant magnétique (1030) comprenant une partie logée à l'intérieur du boîtier en une position disposée latéralement par rapport aux première et seconde régions transparentes à la lumière ; et/ou
le premier composant magnétique (1030) s'étendant de manière circonférentielle autour d'un périmètre externe du boîtier (1004) et le second composant magnétique (1050) comprenant une forme annulaire conçue pour être alignée avec le premier composant magnétique ; et/ou
le premier composant magnétique (1030) comprenant une première partie magnétique (1032) accouplée à une première partie latérale (1020) de boîtier en une position disposée latéralement par rapport aux première et seconde régions transparentes à la lumière.

11. Dispositif d'implant médical, comprenant :
une source de lumière (1170) et un capteur de lumière (1180) ; et
un boîtier (1104) conçu pour recevoir la source de lumière et le capteur de lumière, le boîtier comprenant :
une première partie (1108) de paroi conçue pour être positionnée sur une surface d'une première partie de paroi auriculaire adjacente à une partie de paroi de sinus coronaire, la surface de la partie de paroi auriculaire étant orientée vers une oreillette gauche et la première partie de paroi comprenant une première surface (1110) orientée vers la surface de la partie de paroi auriculaire,
une première région transparente à la lumière (1112) sur la première partie de paroi destinée à permettre le passage à travers celle-ci d'un signal de lumière émis depuis la source de lumière et
une seconde région transparente à la lumière (1114) sur la première partie de paroi destinée à permettre le passage à travers celle-ci d'un signal de lumière réfléchie depuis le sinus coronaire pour une détection par le capteur de lumière.

12. Dispositif selon la revendication 11, comprenant en outre un ancrage (1102) associé à une partie de boîtier disposée latéralement par rapport à la première région transparente à la lumière et à la seconde région transparente à la lumière et comprenant une partie latérale (1132, 1152) présentant la même orientation que celle de la première surface du boîtier, la partie latérale étant conçue pour s'accoupler mécaniquement à une deuxième partie de paroi auriculaire adjacente à la première partie de paroi auriculaire.

13. Dispositif selon la revendication 12, l'ancrage (1102) comprenant au moins un élément parmi une pince, un tire-bouchon, une barbe et une ouverture conçue pour recevoir un point de couture, conçu pour être fixé à la deuxième partie de paroi auriculaire.

14. Dispositif selon la revendication 12 ou 13 :
la partie latérale comprenant une première partie latérale (1132) d'ancrage accouplée à une première partie (1116) de boîtier disposée latéralement par rapport aux première et seconde régions transparentes à la lumière et comprenant la même orientation que celle de la première surface du boîtier et conçue pour être fixée à une deuxième partie de paroi auriculaire ; et
l'ancrage comprenant une seconde partie latérale (1152) d'ancrage accouplée à une seconde partie (1118) de boîtier disposée latéralement par rapport aux première et seconde régions transparentes à la lumière et comprenant la même orientation que celle de la première surface du boîtier et conçue pour être fixée à une troisième partie de paroi auriculaire,
la première partie (1116) de boîtier et la seconde partie (1118) de boîtier se trouvant en des positions opposées.

15. Dispositif selon la revendication 12 ou 13, l'ancrage (1102) s'étendant circonférentiellement autour du boîtier.
